# EUROPEAN PATENT APPLICATION

(11) **EP 3 196 198 A1**
(43) Date of publication of application: **26.07.2017**
(21) Application number: 15820023.8
(22) Date of filing: 17.09.2015
(51) Int. Cl.: C07D 307/91, C09K 11/06, H01L 51/50

(54) **NOVEL COMPOUND AND ORGANIC ELECTROLUMINESCENCE ELEMENT USING SAME**

(30) Priority: 19.09.2014 JP 2014191805
(71) Applicant: Idemitsu Kosan Co., Ltd, Tokyo 100-8321 (JP)
(72) Inventor: KAWAMURA, Masahiro, Sodegaura-shi Chiba 299-0293 (JP); MIZUKI, Yumiko, Sodegaura-shi Chiba 299-0293 (JP); ITO, Hirokatsu, Sodegaura-shi Chiba 299-0293 (JP); HAKETA, Tasuku, Sodegaura-shi Chiba 299-0293 (JP); HAYAMA, Tomoharu, Sodegaura-shi Chiba 299-0293 (JP); TAKAHASHI, Ryota, Sodegaura-shi Chiba 299-0293 (JP)
(74) Representative: Hoffmann Eitle
(86) International application number: PCT/JP2015/004766
(87) International publication number: WO 2016/042772

(57) **Abstract**

A compound represented by the following formula (1). In the formula (1), at least one of Ar₁ to Ar₄ is a group represented by the following formula (2). In the formula (2), R₁₁ to R₁₈ are independently a single bond that is bonded to N in the formula (1), a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms; among R₁₁ to R₁₈, any adjacent two may be bonded to each other to form a ring; and X₁ is an oxygen atom or a sulfur atom.

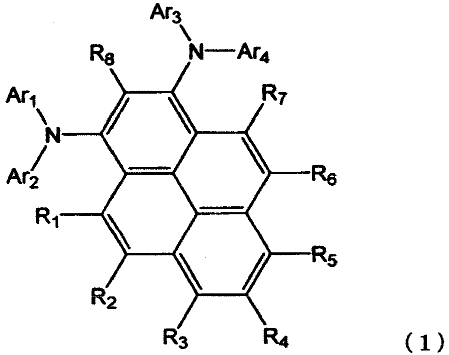

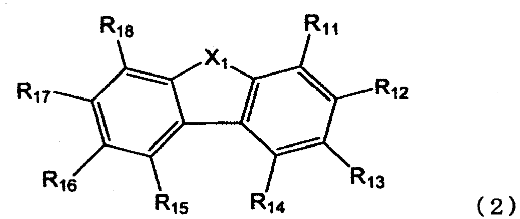

## Description

### Technical Field

The invention relates to a novel compound and an organic electroluminescence device obtained by using the same.

### Background Art

An organic electroluminescence (EL) device is regarded as a promising solid-emitting inexpensive large-area full color display device, and various developments have been conducted so far. In general, an organic EL device comprises an emitting layer and a pair of opposing electrodes that sandwich the emitting layer. When an electrical field is applied between the both electrodes, electrons are injected from the cathode and holes are injected from the anode. Further, these electrons are re-combined with the holes in the emitting layer, create an excited state, and energy is emitted as light when the excited state is returned to the ground state.

In recent years, various materials for an organic EL device have been proposed (for example, Patent Documents 1 and 2). In an organic EL device, further improvement in luminous efficiency, lifetime, color reproducibility or the like has been required. In particular, improvement in color purity (allowing an emission wavelength to be short) is an important factor leading to improvement in color reproducibility regarding a display.

### Related Art Documents

### Patent Documents

Patent Document 1: WO2012/048780
Patent Document 2: WO2013/039221

### Summary of the Invention

An object of the invention is to provide a novel compound capable of allowing an emission wavelength to be short in an organic EL device.

According to one aspect of the invention, the following compound or the like are provided.

A compound represented by the following formula (1): 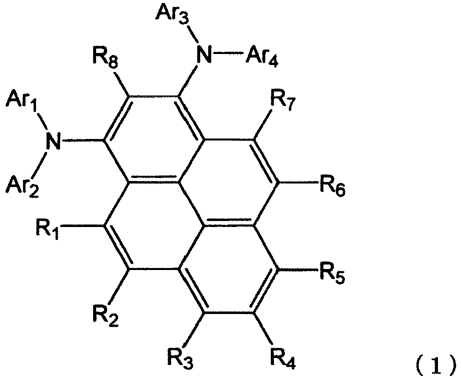
wherein in the formula (1), R₁ to R₈ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 carbon atoms that form a ring (hereinafter referred to as "ring carbon atoms") or a substituted or unsubstituted alkoxy group including 1 to 15 carbon atoms;
at least one of R₁ to R₈ is a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms;
Ar₁ to Ar₄ are independently a substituted or unsubstituted aryl group including 6 to 30 ring carbon atoms or a substituted or unsubstituted heteroaryl group including 5 to 30 atoms that form a ring (hereinafter referred to as "ring atoms"); and
at least one of Ar₁ to Ar₄ is a group represented by the following formula (2): 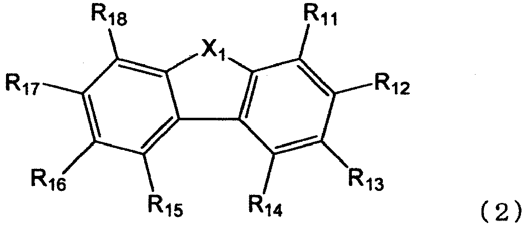
   wherein in the formula (2), R₁₁ to R₁₈ are independently a single bond that is bonded to N in the formula (1), a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms;
   among R₁₁ to R₁₈, any adjacent two may be bonded to each other to form a ring; and
   X₁ is an oxygen atom or a sulfur atom.

According to the invention, it is possible to provide a novel compound that can allow an emission wavelength to be short in an organic EL device.

### Mode for Carrying out the Invention

The compound according to one aspect of the invention is represented by the following formula (1): 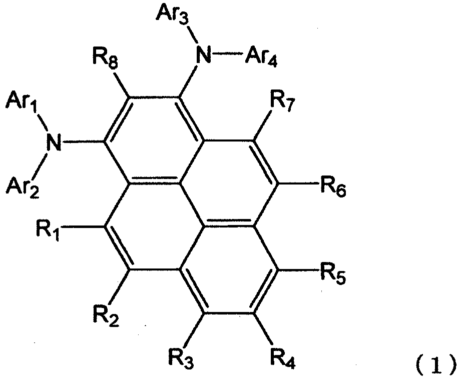
wherein in the formula (1), R₁ to R₈ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms;
at least one of R₁ to R₈ is a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms;
Ar₁ to Ar₄ are independently a substituted or unsubstituted aryl group including 6 to 30 ring carbon atoms or a substituted or unsubstituted heteroaryl group including 5 to 30 ring atoms; and
at least one of Ar₁ to Ar₄ is a group represented by the following formula (2): 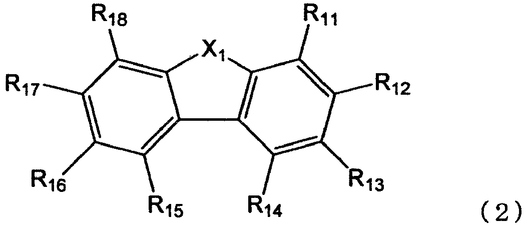
   wherein in the formula (2), R₁₁ to R₁₈ are independently a single bond that is bonded to N in the formula (1), a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms;
   among R₁₁ to R₁₈, any adjacent two may be bonded to each other to form a ring; and
   X₁ is an oxygen atom or a sulfur atom.

Due to the above-mentioned specific structure, a compound according to one aspect of the invention can allow an emission wavelength to be short in an organic EL device.

In the formula (1), it is preferred that at least two of Ar₁ to Ar₄ be a group represented by the formula (2). In this case, at least two groups represented by the formula (2) may be the same as or different from each other.

Further, in the formula (1) it is preferred that Ar₁ and Ar₃ be a group represented by the formula (2). In this case, Ar₁ and Ar₃ may be the same as or different from each other.

In the formula (2), it is preferred that R₁₁ be a single bond that is bonded to N in the formula (1).

In the formula (1), it is preferred that at least one of R₃ to R₅ be a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms. In this case, at least one of R₃ to R₅ is a substituent other than a hydrogen atom.

In the formula (1), it is preferred that R₄ be a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms. In this case, R₄ is a substituent other than a hydrogen atom.

In the formula (1), it is preferred that R₃ and R₅ be independently a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms. In this case, R₃ and R₅ are independently a substituent other than a hydrogen atom.

In the formula (1), at least one of R₁ to R₈ that is a substituent other than a hydrogen atom is preferably a substituted or unsubstituted alkyl group including 1 to 15 carbon atoms, a substituted or unsubstituted alkylsilyl group including 3 to 45 carbon atoms or a substituted or unsubstituted arylsilyl group including 8 to 50 ring carbon atoms, with a substituted or unsubstituted alkyl group including 1 to 6 carbon atoms being more preferable.

In the compound according to one aspect of the invention, it is preferred that R₁₁ be a single bond that is bonded to N in the formula (1) and R₁₈ be a substituted or unsubstituted alkyl group including 1 to 15 carbon atoms, a substituted or unsubstituted alkylsilyl group including 3 to 45 carbon atoms, a substituted or unsubtituted arylsilyl group including 8 to 50 ring carbon atoms, a substituted or unsubstituted aryl group including 6 to 30 ring carbon atoms or a substituted or unsubstituted heteroaryl group including 5 to 30 ring atoms.

Further, it is preferred that the group represented by the formula (2) be a substituent represented by the following formula (3) or the following formula (4): 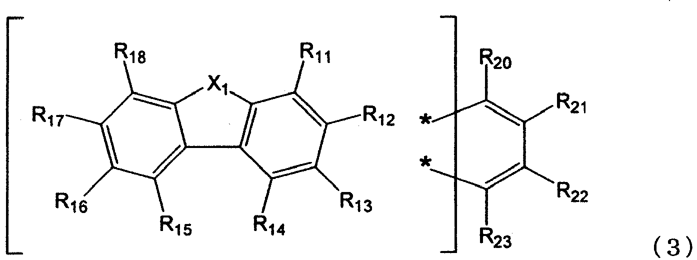
wherein in the formula (3), R₁₁ to R₁₈ and X₁ are the same as defined in the formula (2),
R₂₀ to R₂₃ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms; and
* is a bonding position to any adjacent two of R₁₁ to R₁₈.

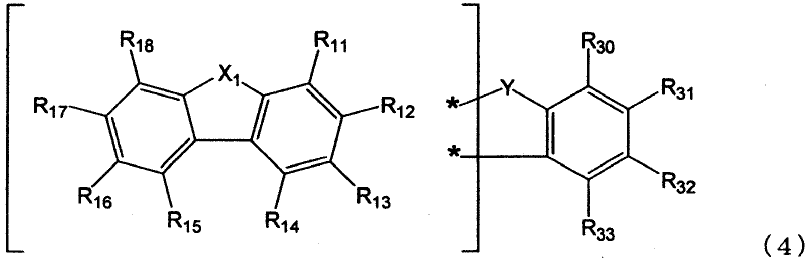

wherein in the formula (4), R₁₁ to R₁₈ and X₁ are the same as defined in the formula (2);
Y is an oxygen atom, a sulfur atom or CR₃₄R₃₅;
R₃₀ to R₃₅ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms; and
* is a bonding position to any adjacent two of R₁₁ to R₁₈.

In the invention, the "hydrogen atom" includes isomers differing in number of neutrons, i.e. protium, deuterium and tritium.

In this specification, ring carbon atoms mean the number of carbon atoms among atoms that constitute a ring itself of a compound having a structure in which atoms or molecules are bonded in a ring form (for example, a monocycle, a fused cycle, an assembly of rings) (for example, monocyclic compounds, fused cyclic compounds, cross-linked compounds, spiro-ring compounds, carbocyclic compounds, heteroaryl compounds). When the ring is substituted by a substituent, carbon atoms included in the substituent is not included in the ring carbon atoms. As for the "ring carbon atoms" mentioned below, the same is applied otherwise unless indicated. For example, a benzene ring has 6 ring carbon atoms, a naphthalene ring has 10 ring carbon atoms, a pyridinyl group has 5 ring carbon atoms, and a furanyl group has 4 ring carbon atoms. Further, when a benzene ring or a naphthalene ring is substituted with an alkyl group as a substituent, for example, the number of carbon atoms in the alkyl group is not included in the ring carbon atoms. Further, when a fluorene ring is bonded, as a substituent, to a fluorene ring, for example (including a spiro-fluorene ring), the number of carbon atoms of a fluorene ring as a substituent is not included in the ring carbon atoms.

In the specification, ring atoms mean the number of atoms that constitute a ring itself of a compound having a structure in which atoms or molecules are bonded in a ring form (for example, a monocycle, a fused cycle, an assembly of rings) (for example, monocyclic compounds, fused cyclic compounds, cross-linked compounds, carbocyclic compounds, heteroaryl compounds). Atoms that do not constitute a ring (for example, a hydrogen atom that terminates an atomic bonding of atoms that constitute a ring) or an atom included in a substituent when the ring is substituted by a substituent are not included in the ring atoms. The same can be applied to the "ring atoms" mentioned below unless otherwise indicated. For example, a pyridine ring has 6 ring atoms, a quinazoline ring has 10 ring atoms, and a furan ring has 5 ring atoms. Hydrogen atoms that are independently bonded to carbon atoms of a pyridine ring or a quinazoline ring or atoms that constitute a substituent (including a spirofluorene ring) are not included in the number of ring atoms. Further, if a fluorene ring is bonded to a fluorene ring as a substituent, for example, the number of atoms of a fluorene ring as a substituent is not included in the number of ring atoms.

The "XX to YY carbon atoms" in the "substituted or unsubstituted ZZ group including XX to YY carbon atoms" means the number of carbon atoms in the ZZ group which is unsubstituted, and does not include the number of carbon atoms of a substituent when the ZZ group is substituted. Here, the "YY" is larger than "XX" and "XX" and "YY" are independently an integer of 1 or more.

The "XX to YY" atoms" in the "a substituted or unsubstituted ZZ group including XX to YY atoms" means the number of atoms in the ZZ group which is unsubstituted, and does not include the number of atoms of a substituent when the ZZ group is substituted. Here, the "YY" is larger than "XX", and "XX" and "YY" are independently an integer of 1 or more.

The "unsubstituted" in the "substituted or unsubstituted..." means that a group or an atom is not substituted by the above-mentioned substituent and a hydrogen atom is bonded thereto.

A detailed explanation will be given below on each group represented by each of the above formulas and a substituent in the "substituted or unsubstituted...".

As the halogen atom, fluorine, chlorine, bromine, iodine or the like can be given, with a fluorine atom being preferable.

As the alkyl group, a methyl group, an ethyl group, a propyl group, an isopropyl group, a n-butyl group, a s-group, an isobutyl group, a t-butyl group, a n-pentyl group, a n-hexyl group, a n-heptyl group, a n-octyl group or the like can be given.

The number of carbon atoms in the alkyl group is preferably 1 to 10, and more preferably 1 to 6. Among these, a methyl group, an ethyl group, a propyl group, an isopropyl group, a n-butyl group, a s-butyl group, an isobutyl group, a t-butyl group, a n-pentyl group and a n-hexyl group are preferable.

The alkyl group may be cyclic. As a cyclic alkyl group, a cyclopropyl group, a cyclobutyl group, a cyclopentyl group, a cyclohexyl group, a 4-methylcyclohexyl group, an adamantly group, a norbornyl group or the like can be given. The ring carbon atoms is preferably 3 to 10, with 5 to 8 being further preferable.

As the aryl group, a phenyl group, a 1-naphthyl group, 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a naphthacenyl group, a pyrenyl group, a chrysenyl group, a benzo[c]phenanthryl group, a benzo[g]chrysenyl group, a triphenylenyl group, a 1-fluorenyl group, a 2-fluorenyl group, a 3-fluorenyl group, a 4-fluorenyl group, a 9-fluorenyl group, a benzofluorenyl group, a dibenzofluorenyl group, a 2-biphenylyl group, a 3-biphenylyl group, a 4-biphenylyl group, a terphenyl group, a fluoranthenyl group or the like can be given, for example.

It is preferred that the aryl group have 6 to 20, more preferably 6 to 12, ring carbon atoms. Among the above-mentioned aryl groups, a phenyl group, a biphenyl group, a tolyl group, a xylyl group and a 1-naphthyl group are particularly preferable.

The alkylsilyl group is represented by -SiY₃, and as examples of Y, the examples of the alkyl group given above can be given. As the alkylsilyl group, a trimethylsilyl group, a triethylsilyl group, a t-butyldimethylsilyl group, a vinyldimethylsilyl group, a propyldimethylsilyl group, a triisopropylsilyl group or the like can be given.

The arylsilyl group is a silyl group substituted by 1 to 3 aryl group(s). As examples of the aryl group, examples of the aryl group mentioned above can be given. The above-mentioned alkyl group may be substituted by the alkyl group mentioned above in addition to the aryl group. As the arylsilyl group, a triphenylsilyl group or the like can be given.

The alkoxy group is represented by -OY. As examples of Y, examples of the alkyl group mentioned above can be given. The alkoxy group is a methoxy group or an ethoxy group, for example.

The aryloxy group is represented by -OZ. As examples of Z, examples of the aryl group mentioned above can be given. The aryloxy group is a phenoxy group, for example.

The alkylthio group is represented by -SY. As examples of Y, examples of the alkyl group mentioned above can be given.

The arylthio group is represented by -SZ. As examples of Z, examples of the aryl group mentioned above can be given.

As the heteroaryl group, a pyrrolyl group, a pyrazinyl group, a pyridinyl group, an indolyl group, an isoindolyl group, an imidazolyl group, a furyl group, a benzofuranyl group, an isobenzofuranyl group, a 1-dibenzofuranyl group, a 2-dibenzofuranyl group, a 3-dibenzofuranyl group, a 4-dibenzofuranyl group, a 1-dibenzothiophenyl group, a 2-dibenzothiophenyl group, a 3-dibenzothiophenyl group, a 4-dibenzothiophenyl group, a quinolyl group, an isoquinolyl group, a quinoxalinyl group, a 1-carbazolyl group, a 2-carbazolyl group, a 3-carbazolyl group, a 4-carbazolyl group, a 9-carbazolyl group, a phenanthridinyl group, an acridinyl group, a phenanthrolinyl group, a phenazinyl group, a phenothiazinyl group, a phenoxazinyl group, an oxazolyl group, an oxadiazolyl group, a furazanyl group, a thienyl group, a benzothiophenyl group or the like can be given.

The ring atoms of the heteroaryl group is preferably 5 to 20, with 5 to 14 being further preferable. A 1-dibenzofuranyl group, a 2-dibenzofuranyl group, a 3-dibenzofuranyl group, a 4-dibenzofuranyl group, a 1-dibenzothiophenyl group, a 2-dibenzothiophenyl group, a 3-dibenzothiophenyl group, a 4-dibenzothiophenyl group, a 1-carbazolyl group, a 2-carbazolyl group, a 3-carbazolyl group, a 4-carbazolyl group and a 9-carbazolyl group are preferable.

The above-mentioned "carbazolyl group" includes the following structures.

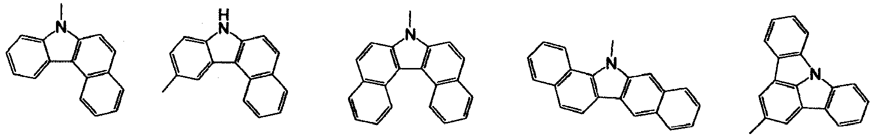

The above-mentioned heteroaryl group includes the following structure. 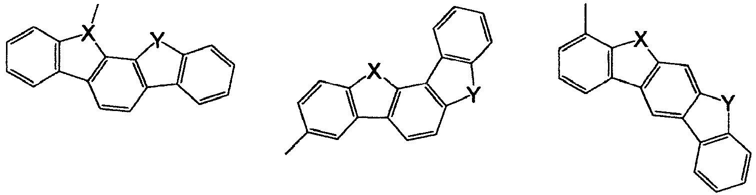 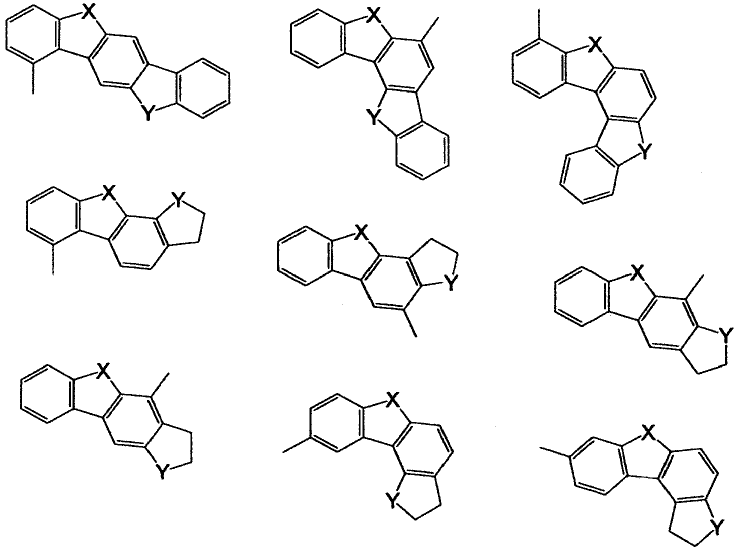 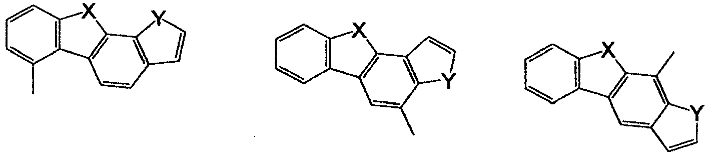 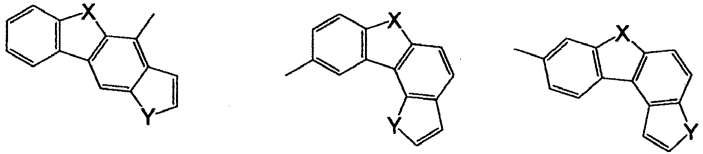 wherein in the formula, X and Y are independently an oxygen atom, a sulfur atom, a nitrogen atom or a - NH- group.

A ring that can be formed by any two that are adjacent among R₁₁ to R₁₈ in the formula (1) is a saturated ring, an unsaturated ring or an aromatic ring. As the ring, a benzene ring, a naphthalene ring, a phenanthrene ring, an indene ring, an indole ring, a furan ring, a thiophene ring, a benzoindene ring, a benzoindole ring, a benzofuran ring, a benzothiophene ring or the like can be given.

As the substituent of each of the above-mentioned group in the "substituted or unsubstituted... ", in addition to the halogen atom, the cyano group, the alkyl group, the alkylsilyl group, the arylsilyl group, the alkoxy group, the aryl group, the heteroaryl group, the aryloxy group, the alkylthio group, the arylthio group mentioned above, a hydroxyl group, a nitro group, a carboxy group or the like can be given.

These substituents may be further substituted by the above-mentioned substituents. Further, these substituents may be bonded to each other to form a ring.

Examples of a compound according to one aspect of the invention are given below.

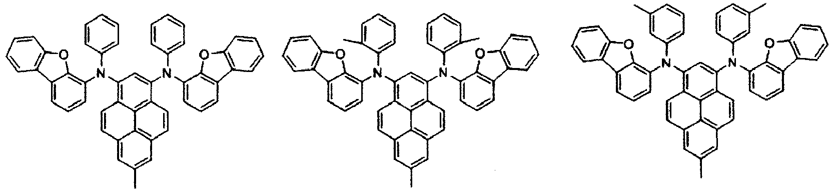

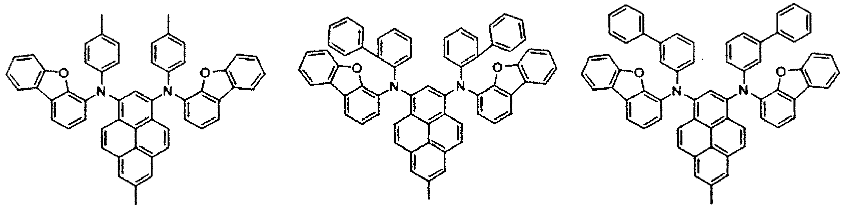

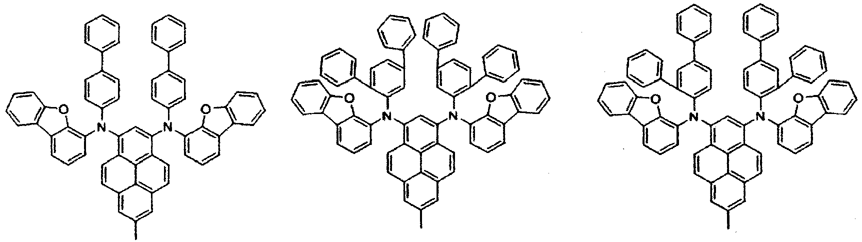

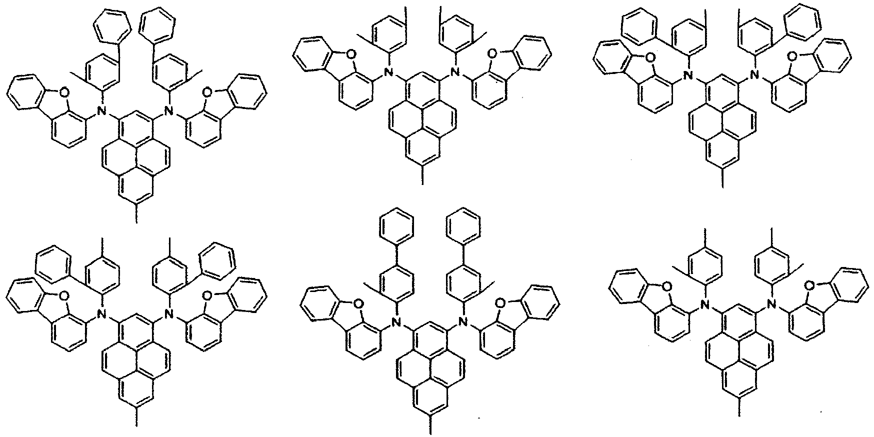

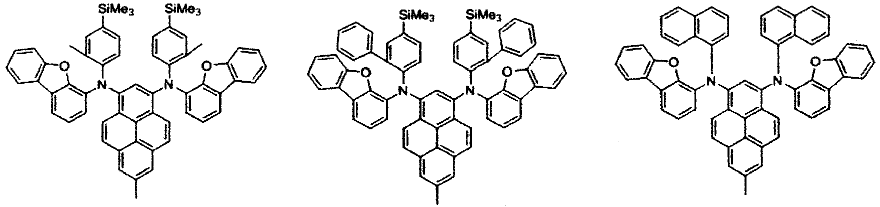

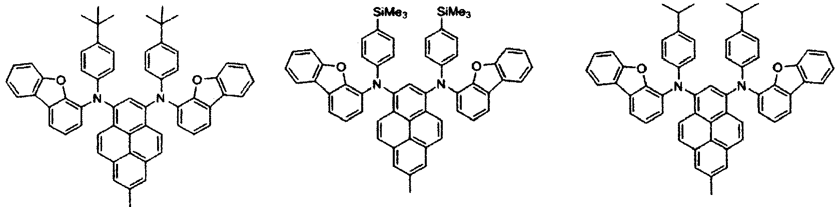

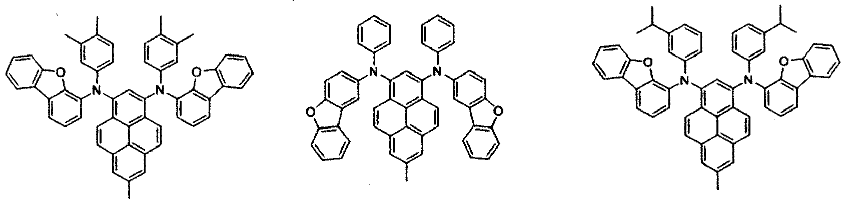

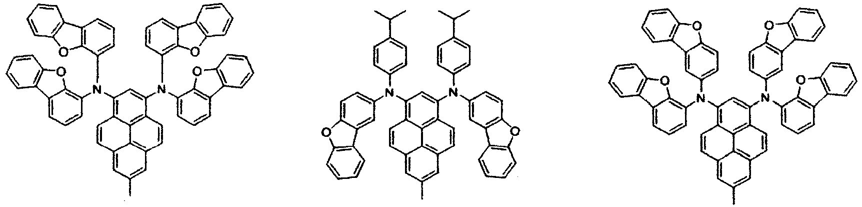

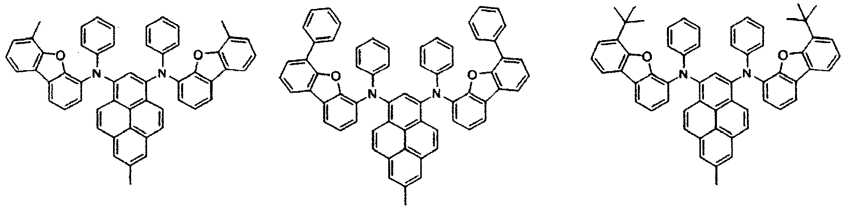

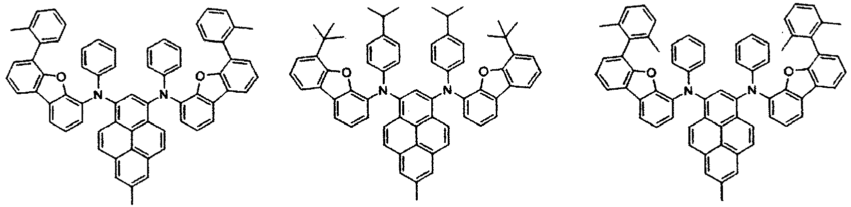

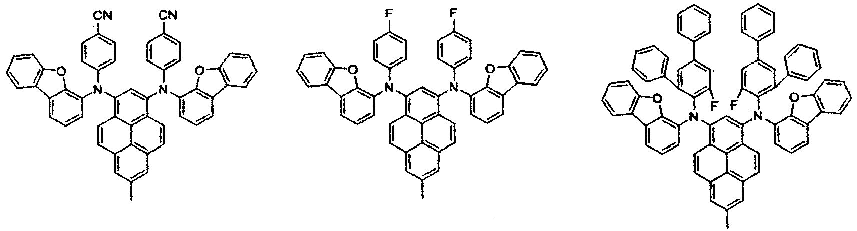

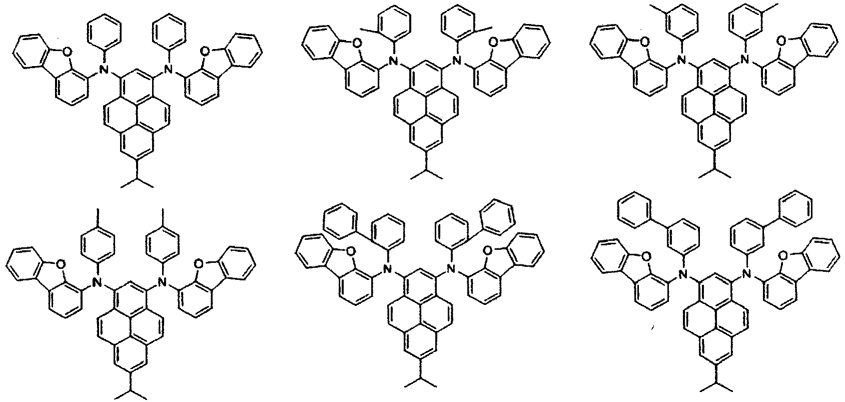

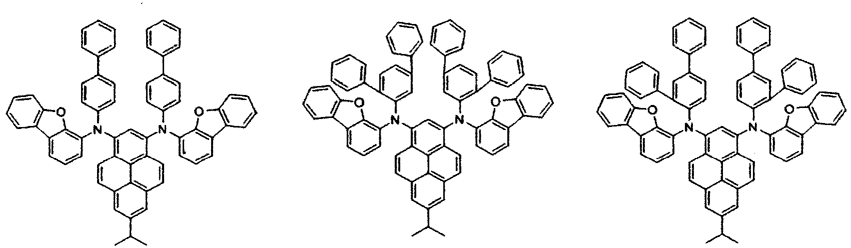

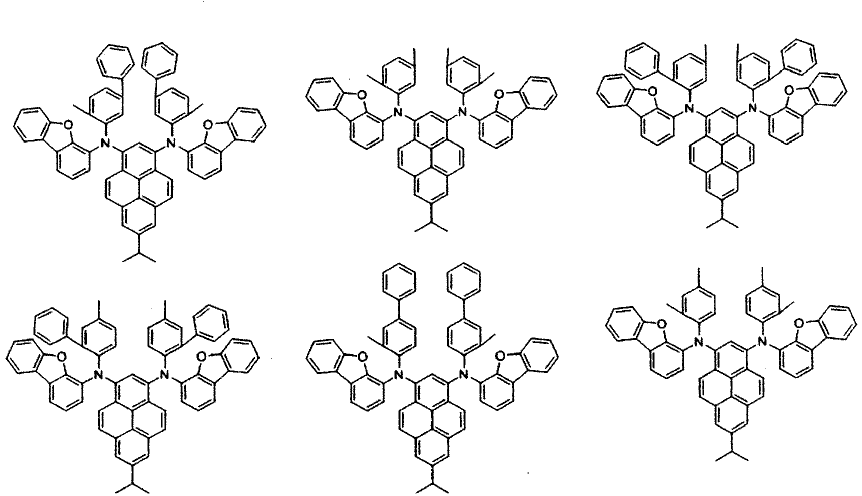

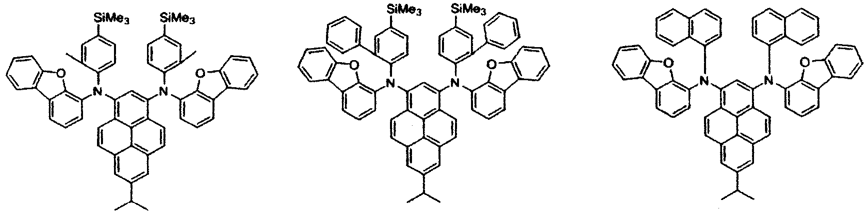

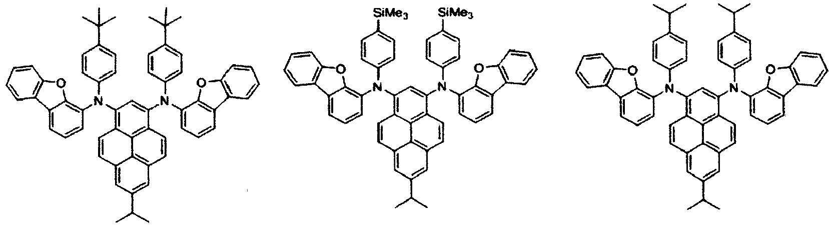

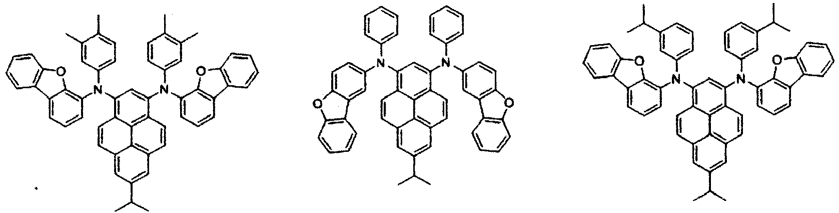

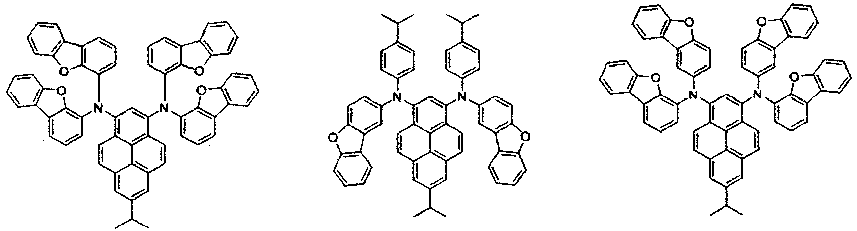

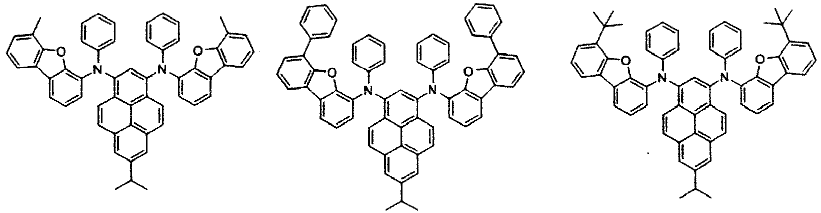

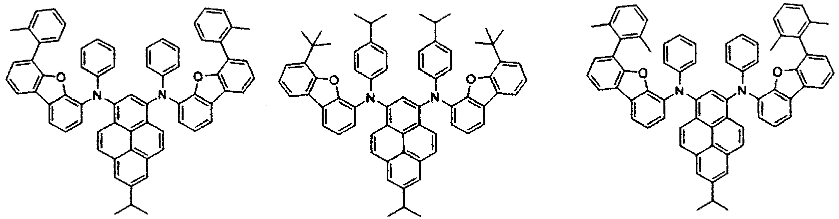

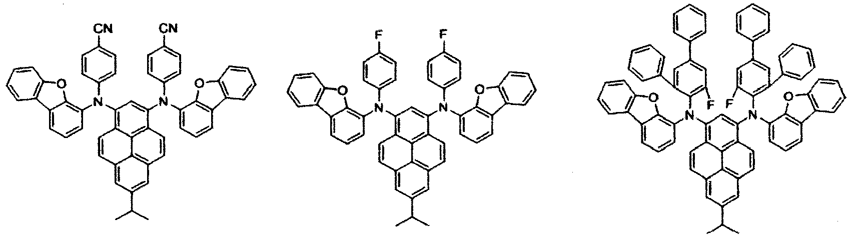

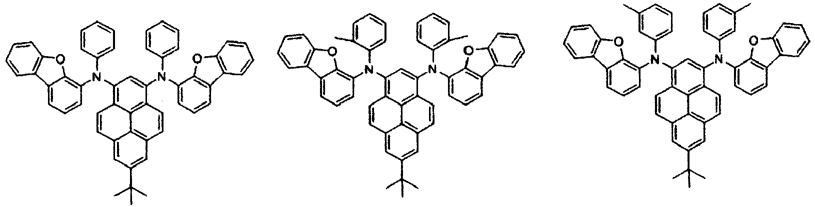

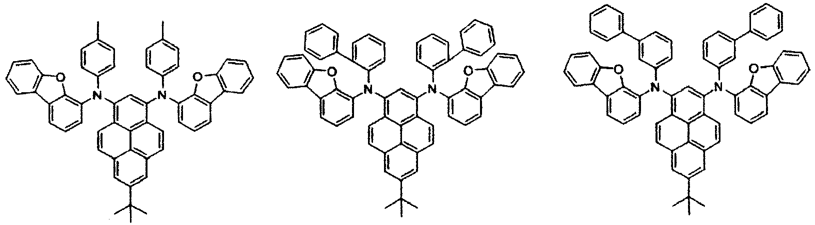

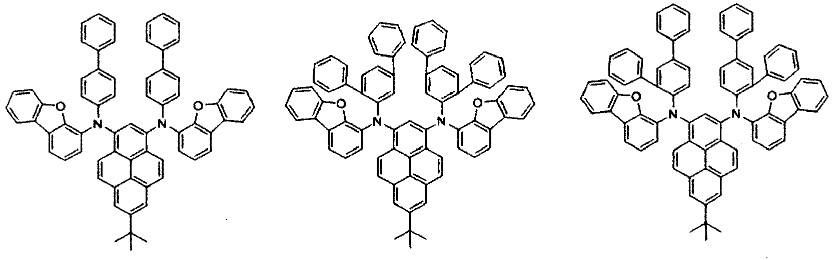

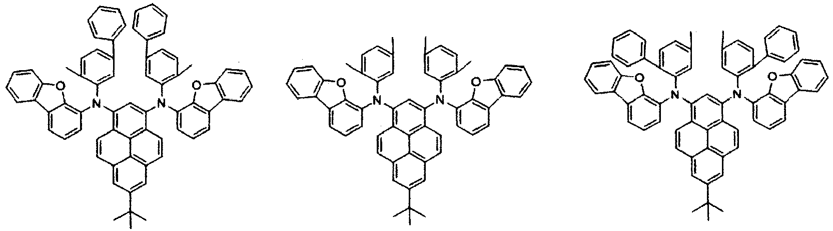

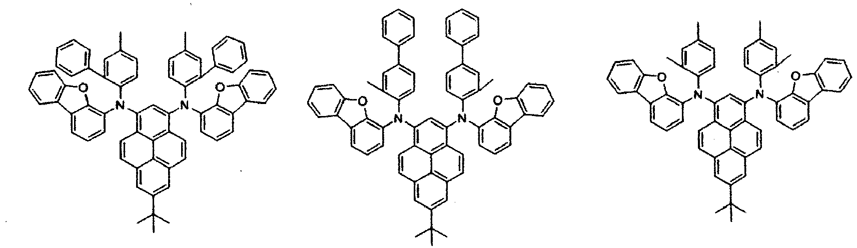

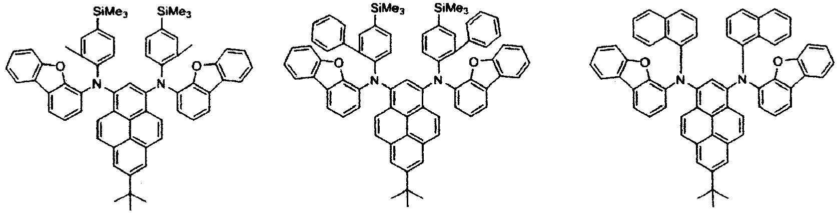

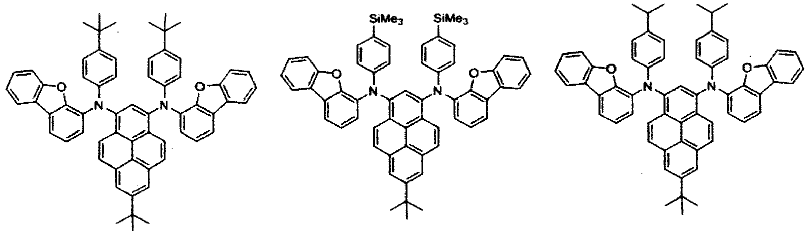

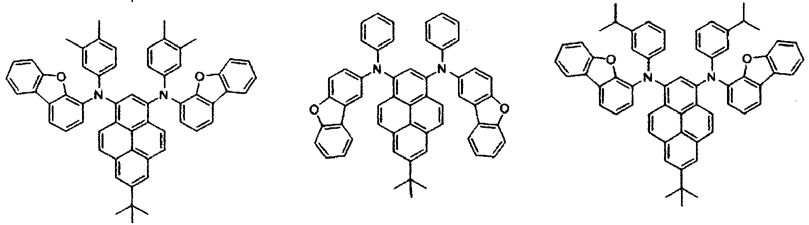

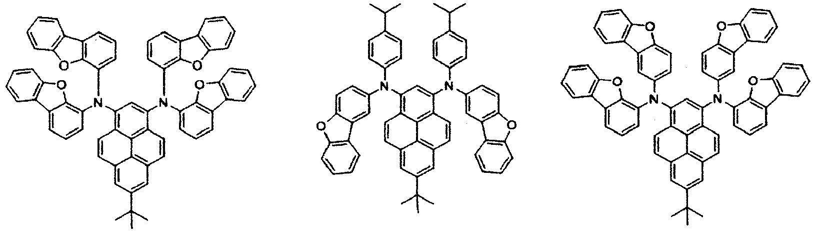

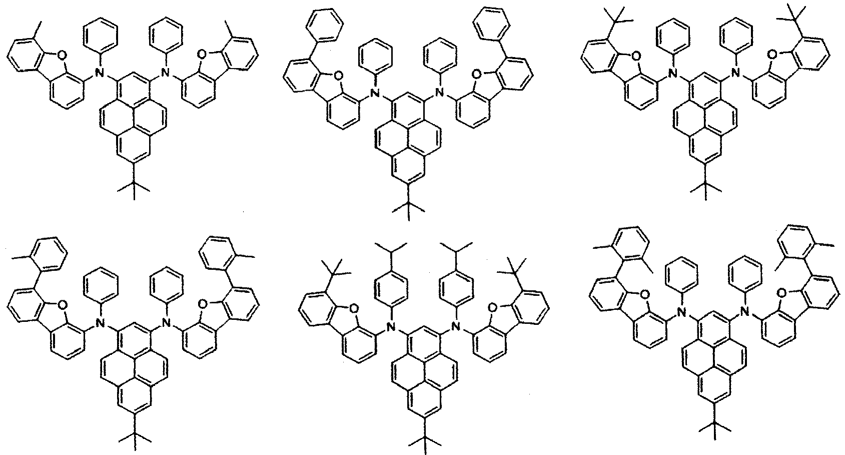

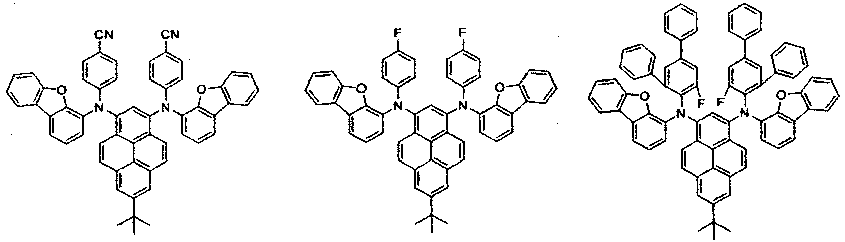

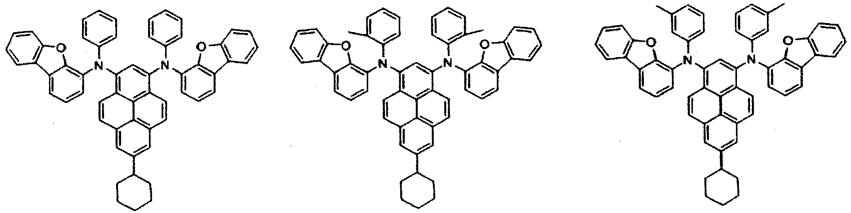

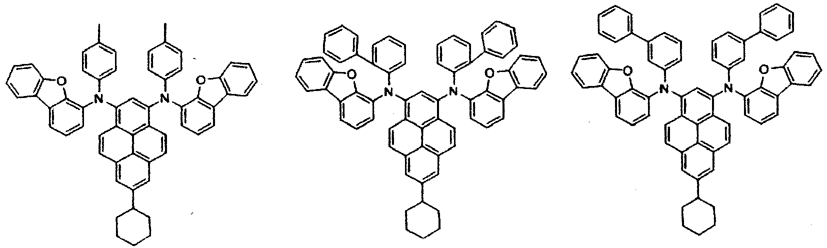

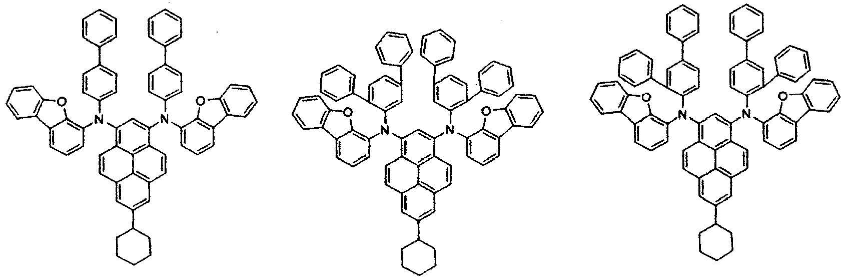

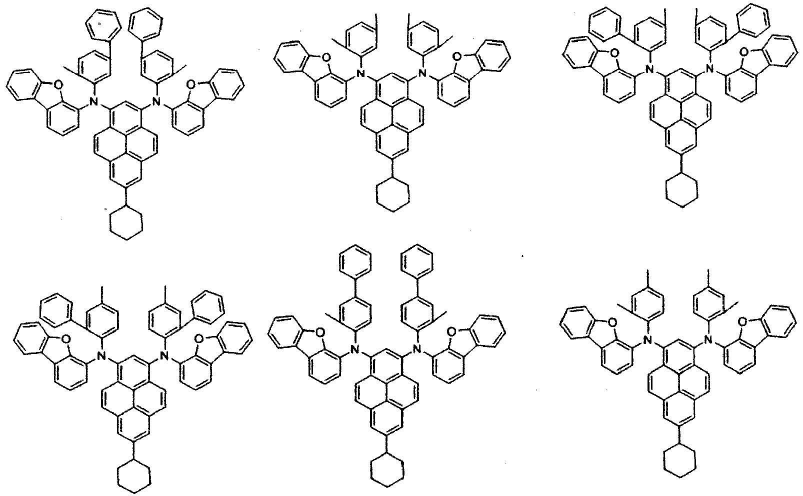

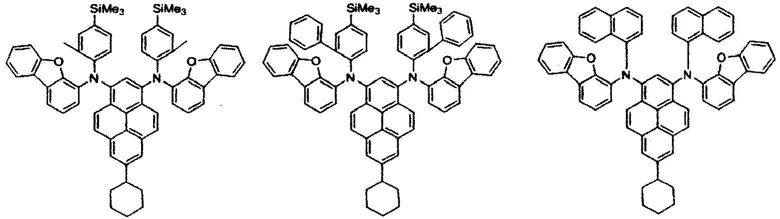

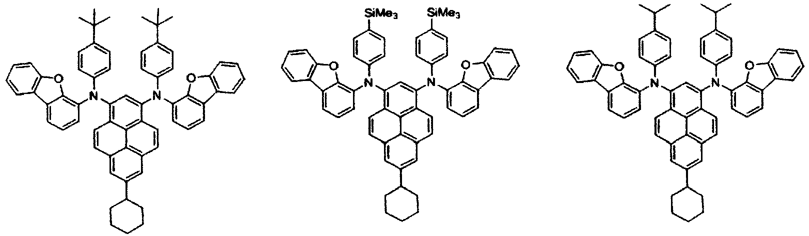

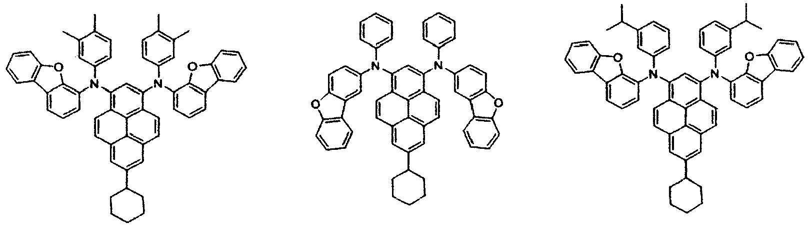

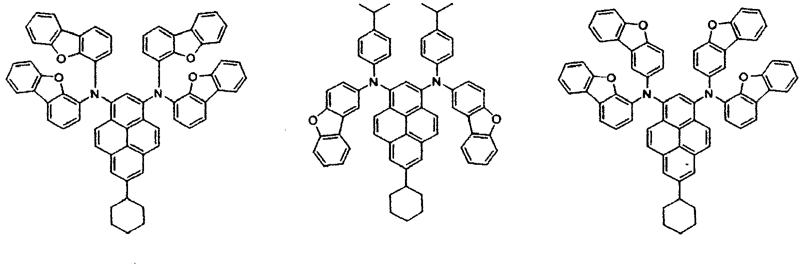

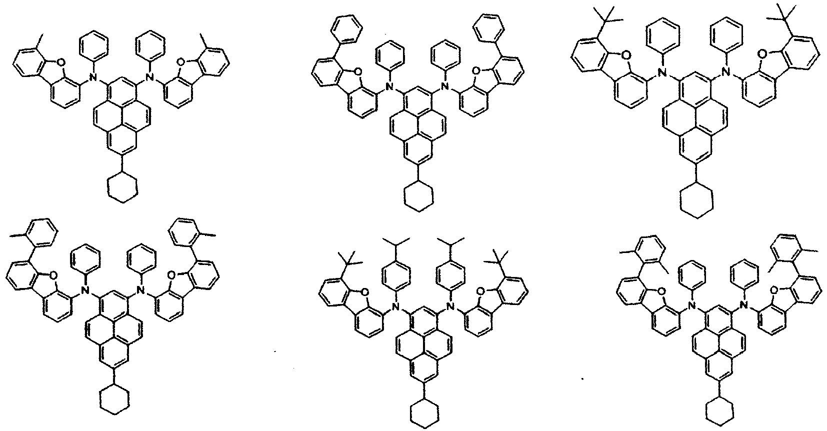

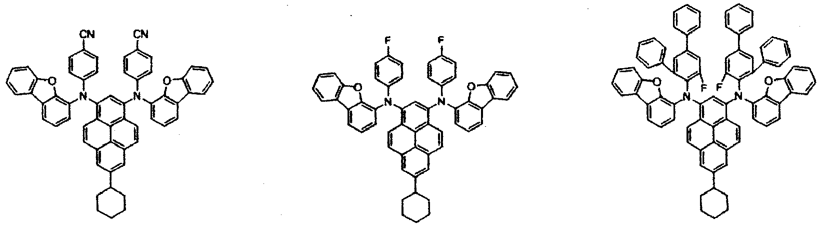

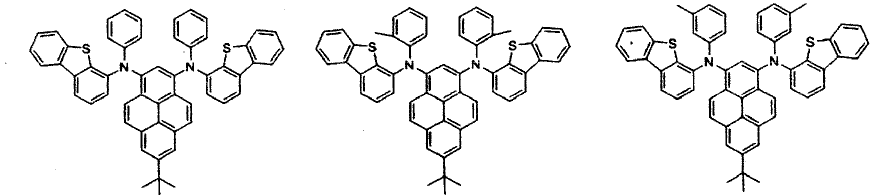

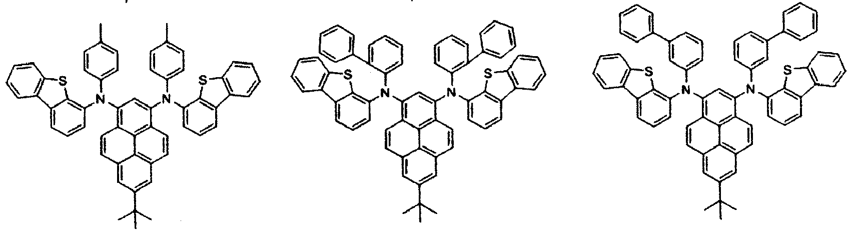

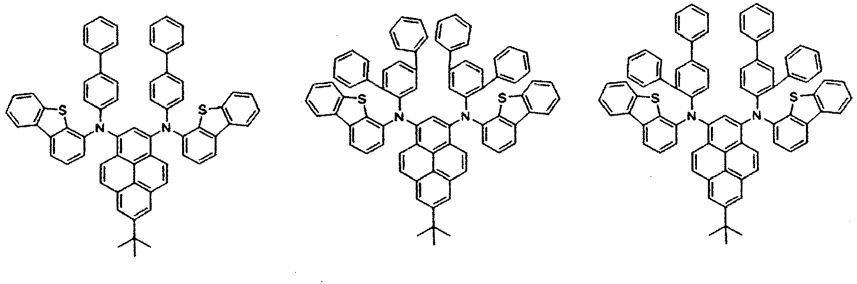

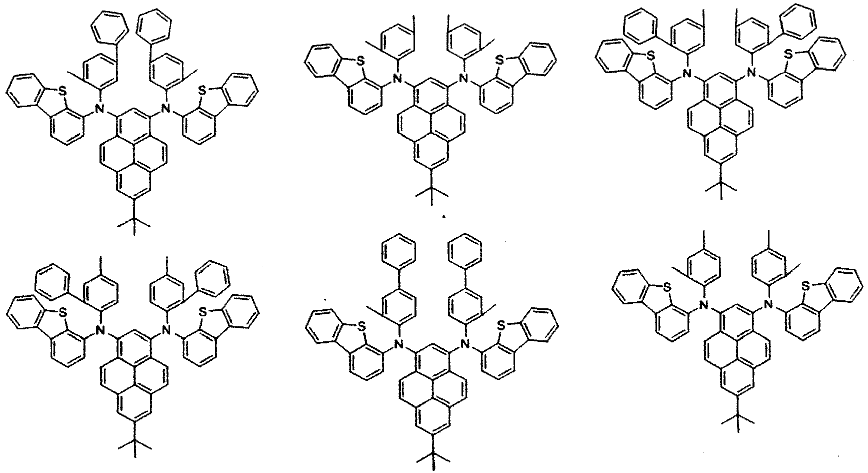

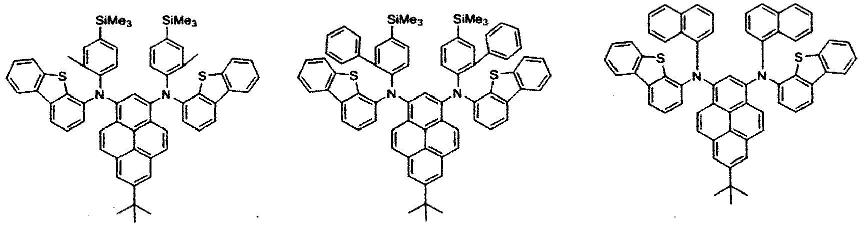

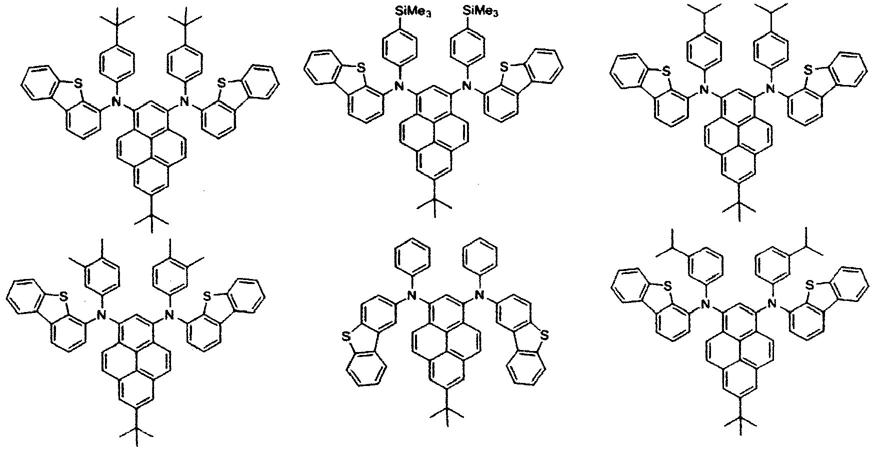

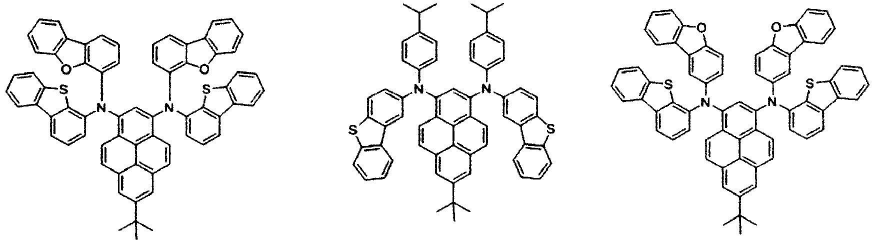

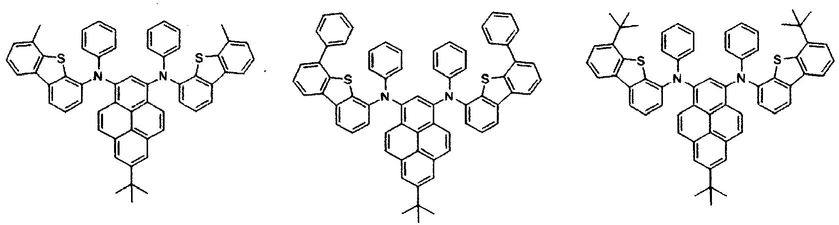

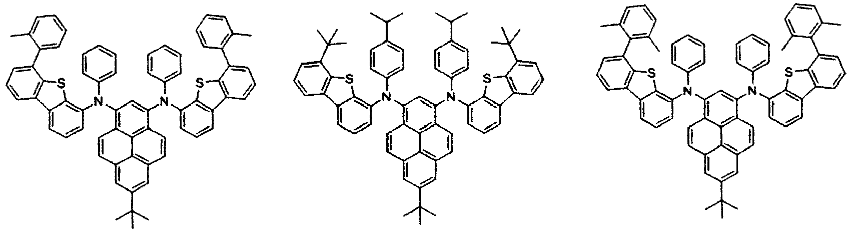

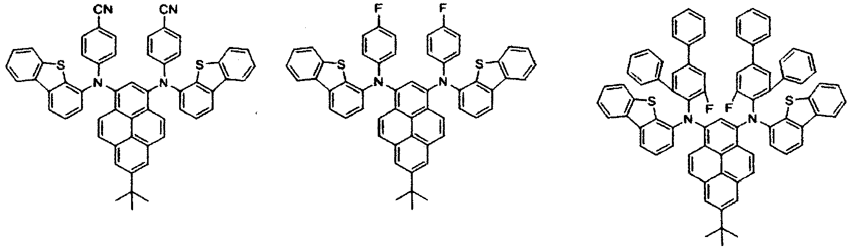

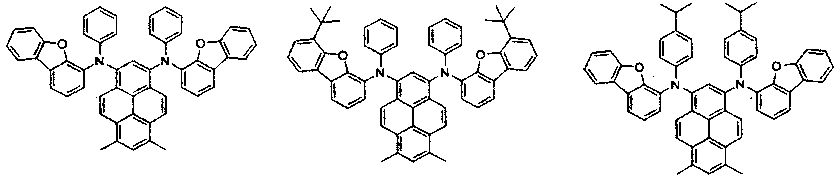

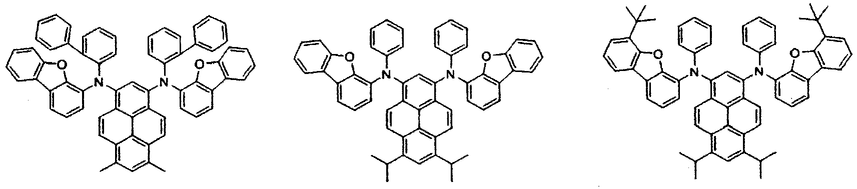

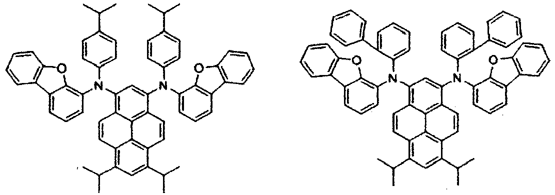

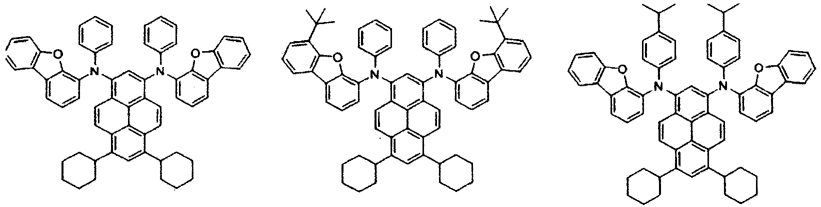

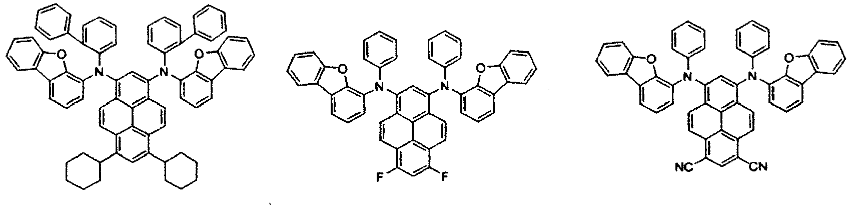

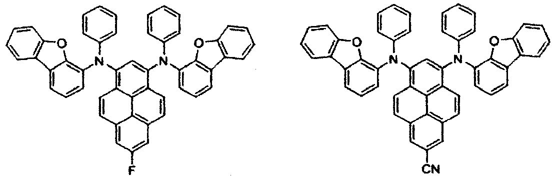

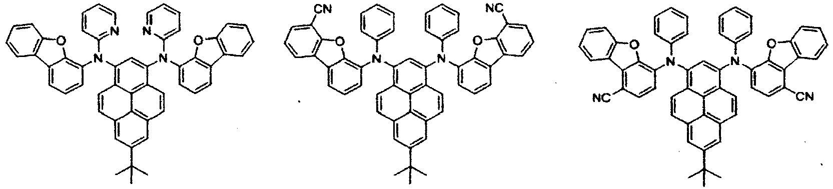

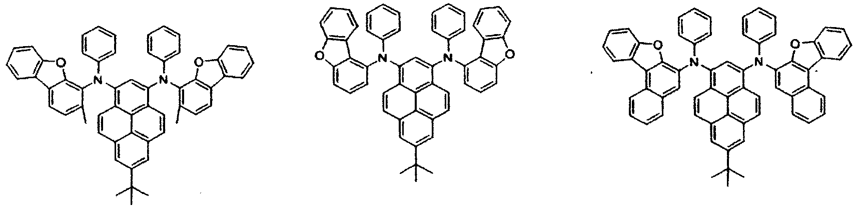

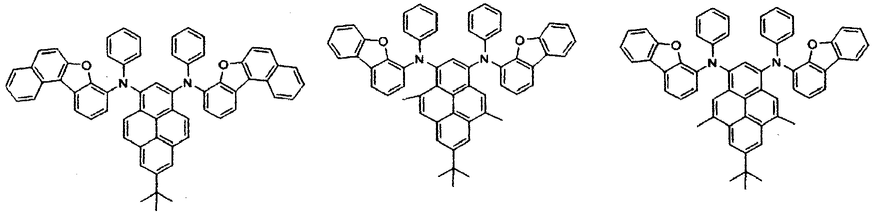

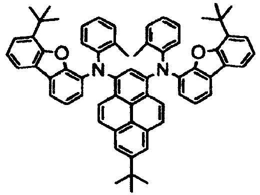

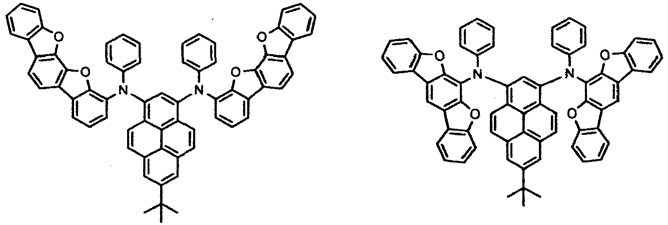

The compound as one aspect of the invention can be synthesized in accordance with the reactions explained in the examples, and by using known alternative reactions or raw materials suited to an intended product.

The compound as one aspect of the invention can be used as a material for an organic electroluminescence device, and is preferably an emitting material for an organic electroluminescence device, and more preferably a dopant material for an organic electroluminescence device.

The organic EL device according to one aspect of the invention comprises a cathode and an anode, and one or more organic thin film layers including at least an emitting layer disposed between the cathode and the anode and at least one layer of the organic thin film layers comprises the compound as one aspect of the invention singly or as a component of a mixture.

The organic EL device as one aspect of the invention can allow an emission wavelength to be short due to presence of the compound that has the specific structure mentioned above in the organic thin film layers.

It is preferred that, in the organic EL device as one aspect of the invention, the emitting layer thereof comprises the compound as one aspect of the invention. At this time, the emitting layer may be composed only of the above-mentioned compound. Alternatively, the emitting layer may comprise the above-mentioned compound as a host material or as a dopant material.

In one aspect of the invention, it is preferred that the above-mentioned compound be a dopant material.

### (Host material)

As the host material of the emitting layer, an anthracene derivative, a polycyclic aromatic skeleton-containing compound or the like can be given. An anthracene derivative is preferable.

As the host material of the emitting layer, an anthracene derivative represented by the following formula (5) can be used, for example.

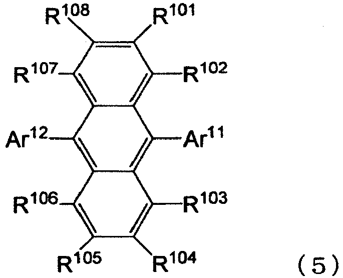

In the formula (5), Ar¹¹ and Ar¹² are independently a substituted or unsubstituted monocyclic ring group including 5 to 50 ring atoms or a substituted or unsubstituted fused cyclic group including 8 to 50 ring atoms.

R¹⁰¹ to R¹⁰⁸ are independently a group selected from the group consisting of a hydrogen atom, a substituted or unsubstituted monocyclic ring group including 5 to 50 (preferably 5 to 30, more preferably 5 to 20, and further preferably 5 to 12) ring atoms, a substituted or unsubstituted fused cyclic group including 8 to 50 (preferably 8 to 30, more preferably 8 to 20 and further preferably 8 to 14) ring atoms, a group composed of a combination of the monocyclic ring group and the fused cyclic group, a substituted or unsubstituted alkyl group including 1 to 50 (preferably 1 to 20, more preferably 1 to 10 and further preferably 1 to 6) carbon atoms, a substituted or unsubstituted cycloalkyl group including 3 to 50 (preferably 3 to 20, more preferably 3 to 10 and further preferably 5 to 8) ring carbon atoms, a substituted or unsubstituted alkoxy group including 1 to 50 (preferably 1 to 20, more preferably 1 to 10 and further preferably 1 to 6) carbon atoms, a substituted or unsubstituted aralkyl group including 7 to 50 (preferably 7 to 20, more preferably 7 to 14) ring carbon atoms, a substituted or unsubstituted aryloxy group including 6 to 50 (preferably 6 to 20, more preferably 6 to 12) ring carbon atoms, a substituted or unsubstituted silyl group, a halogen atom, and a cyano group.

It is preferred that all of R¹⁰¹ to R¹⁰⁸ be a hydrogen atom or one of R¹⁰¹ and R¹⁰⁸, one of R¹⁰⁴ and R¹⁰⁶, both of R¹⁰¹ and R¹⁰⁶ or both of R¹⁰⁸ and R¹⁰⁴ be a group selected from the group consisting of a monocyclic ring group including 5 to 50 ring atoms (preferably a phenyl group, a biphenyl group, a terphenyl group), a substituted or unsubstituted alkyl group including 1 to 50 carbon atoms (preferably a methyl group, an ethyl group, a n-propyl group, an isopropyl group, a n-butyl group, an isobutyl group, a s-butyl group, a t-butyl group) and a substituted silyl group (preferably a trimethylsilyl group). It is more preferred that all of R¹⁰¹ to R¹⁰⁸ be a hydrogen atom.

The monocyclic ring group in the formula (5) is a group composed only of a ring structure that does not have a fused cyclic structure.

As specific examples of the monocyclic ring group including 5 to 50 ring atoms, an aromatic group such as a phenyl group, a biphenyl group, a terphenylyl group and a quaterphenyl group and a heterocylic group such as a pyridiyl group, a pyrazyl group, a pyrimidyl group, a triazinyl group, a furyl group and a thienyl group are preferable.

Among these, a phenyl group, a biphenyl group and a terphenylyl group are preferable as the monocyclic ring group.

The fused cyclic group in the formula (5) is a group obtained by fusing of two or more ring structures.

As specific examples of a fused cyclic ring including 8 to 50 ring atoms, a fused aromatic ring group such as a naphthyl group, a phenanthryl group, an anthryl group, a chrysenyl group, a benzoanthryl group, a benzophenanthryl group, a triphenylenyl group, a benzochrycenyl group, an indenyl group, a fluorenyl group, a benzofluorenyl group, a dibenzofluorenyl group, a fluoranthenyl group, and a benzofluoranthenyl group or a fused heterocyclic group such as a benzofuranyl group, a benzothiophenyl group, an indolyl group, a dibenzofuranyl group, a dibenzothiophenyl group, a carbazolyl group, a quinolyl group and a phenanthrolinyl group are preferable.

The fluorenyl group mentioned above may have one or two substituents at the 9^{th} position. As the substituent, for example, an alkyl group, an aryl group, an alkylsilyl group, an arylsilyl group, an alkoxy group or the like can be given. As specific examples of the fluorenyl group, a 9,9-dimethylfluorenyl group, a 9,9-diphenylfluorenyl group or the like can be given, for example. Hereinafter, when referring to the "fluorenyl group", the fluorenyl group may have the similar substituents unless otherwise indicated.

Among them, a naphthyl group, a phenanthryl group, an anthryl group, a fluorenyl group (specifically, a 9,9-dimethylfluorenyl group, etc.), a fluoranthenyl group, a benzanthryl group, a dibenzothiophenyl group, a dibenzofuranyl group and a carbazolyl group are preferable as the fused cyclic group.

Specific examples of the alkyl group, the cycloalkyl group (cyclic alkyl group), the alkoxy group, an alkyl part and an aryl part of the aralkyl group, the aryloxy group, the substituted silyl group (alkylsilyl group, arylsilyl group) and the halogen atom in the formula (5) are the same as each group in the formula (1) and the specific examples of the substituent in the "substituted or unsubstituted...".

The aralkyl group is represented by -Y-Z. As examples of Y, examples of an alkylene corresponding to the examples of the alkyl group mentioned above can be given. As examples of Z, examples of the aryl group mentioned above can be given. The aralkyl group is preferably an aralkyl group including 7 to 50 carbon atoms (an aryl part includes 6 to 49 (preferably 6 to 30, more preferably 6 to 20, and particularly preferably 6 to 12) carbon atoms and an alkyl part includes 1 to 44 (preferably 1 to 30, more preferably 1 to 20, further preferably 1 to 10, and particularly preferably 1 to 6)) carbon atoms. For example, the aralkyl group is a benzyl group, a phenylethyl group and a 2-phenylpropan-2-yl group.

As the preferable substituent in the "substituted or unsubstituted" in Ar¹¹, Ar¹² and to R⁸, a monocyclic ring group, a fused cyclic group, an alkyl group, a cycloalkyl group, a silyl group, an alkoxy group, a cyano group and a halogen atom (in particular, fluorine) are preferable. A monocyclic ring group and a fused cyclic group are particularly preferable. Preferable specific substituents are as mentioned above.

As the substituent of Ar¹¹ and Ar¹², the monocyclic ring group or the fused cyclic group mentioned above is preferable.

It is preferred that the anthracene derivative represented by the formula (5) be any one of the following anthracene derivatives (A), (B) and (C), and a suitable anthracene derivative is selected according to the constitution or required properties of an organic EL device to which the anthracene derivative is applied.

### Anthracene derivative (A)

The anthracene derivative (A) is an anthracene derivative represented by the formula (5) in which Ar¹¹ and Ar¹² are independently a substituted or unsubstituted fused cyclic group including 8 to 50 ring atoms. As such anthracene derivative, Ar¹¹ and Ar¹² may be the same as or different from each other.

An anthracene derivative that is a substituted or unsubstituted anthracene derivative represented by the formula (5) in which Ar¹¹ and Ar¹² differ (including a case where the positions to which an anthracene ring is bonded differ) is particularly preferable. Specific preferable examples of the fused cycle are as mentioned above. Among these, a naphthyl group, a phenanthryl group, a benzanthryl group, a fluorenyl group (specifically, a 9,9-dimethylfluorenyl group, or the like), a dibenzofuranyl group are preferable.

### Anthracene derivative (B)

The anthracene derivative (B) is an anthracene derivative represented by the formula (5) in which one of Ar¹¹ and Ar¹² is a substituted or unsubstituted monocyclic ring group including 5 to 50 ring atoms, and the other of Ar¹¹ and Ar¹² is a fused cyclic group including 8 to 50 ring atoms.

As a preferable aspect of the anthracene derivative (B), a derivative in which Ar¹² is a naphthyl group, a phenanthryl group, a benzanthryl group, a fluorenyl group (specifically, a 9,9-dimethylfluorenyl group, etc.) or a dibenzofuranyl group and Ar¹¹ is an unsubstituted phenyl group or a phenyl group substituted by a monocyclic ring group or a fused cyclic group (for example, a phenyl group, a biphenyl group, a naphthyl group, a phenanthryl group, a fluorenyl group (specifically, a 9,9-dimethylfluorenyl group, etc.) or a dibenzofuranyl group) can be given. Specific preferable examples of the monocyclic ring group and the fused cyclic group are as mentioned above.

As another preferable aspect of the anthracene derivative (B), a derivative in which Ar¹² is a substituted or unsubstituted fused cyclic group including 8 to 50 ring atoms and Ar¹¹ is an unsubstituted phenyl group can be given. In this case, as the fused cyclic group, a phenanthryl group, a fluorenyl group (specifically, a 9,9-dimethylfluorenyl group, etc.), a dibenzofuranyl group and a benzanthryl group or the like are particularly preferable.

### Anthracene derivative (C)

The anthracene derivative (C) is an anthracene derivative represented by the formula (5) in which Ar¹¹ and Ar¹² are independently a substituted or unsubstituted monocyclic ring group including 5 to 50 ring atoms.

As a preferable aspect of the anthracene derivative (C), a derivative in which both of Ar¹¹ and Ar¹² are a substituted or unsubstituted phenyl group can be given. As a further preferable aspect, a derivative in which Ar¹¹ is an unsubstituted phenyl group and Ar¹² is a phenyl group substituted with a monocyclic ring group or a fused cyclic group and a derivative in which Ar¹¹ and Ar¹² are independently a phenyl group that is substituted by a monocyclic ring group or a fused cyclic group can be given.

The specific examples of the preferable monocyclic ring group or the preferable fused cyclic group as above-mentioned substituent are as mentioned above. A further preferable monocyclic ring group as the substituent is a phenyl group or a biphenyl group, and a further preferable fused cyclic group as the substituent is a naphthyl group, a phenanthryl group, a fluorenyl group (specifically, a 9,9-dimethylfluorenyl group, or the like), a dibenzofuranyl group or a benzanthryl group.

As specific examples of the anthracene derivative represented by the formula (5), the following derivatives can be given.

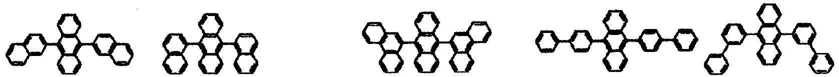

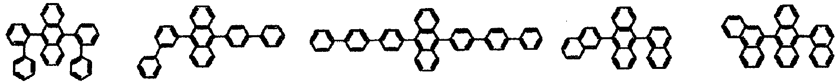

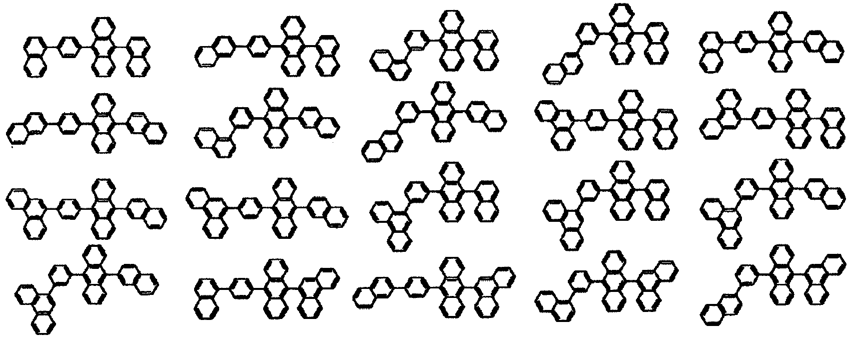

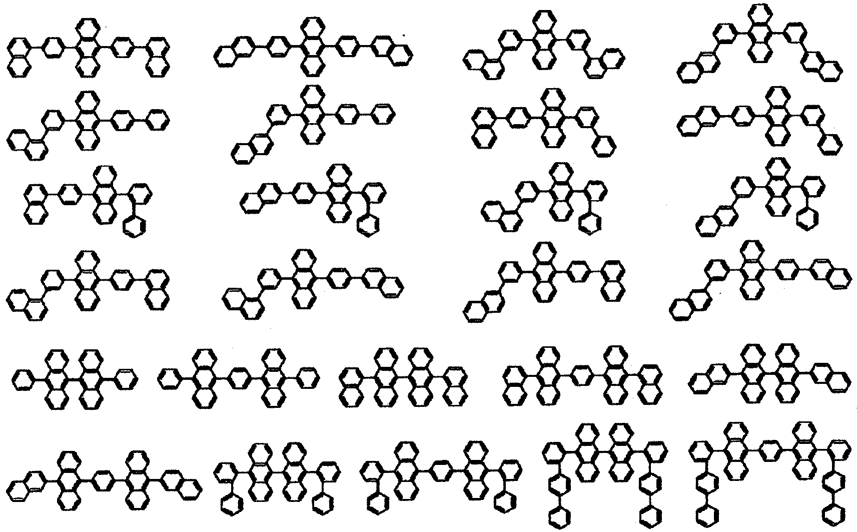

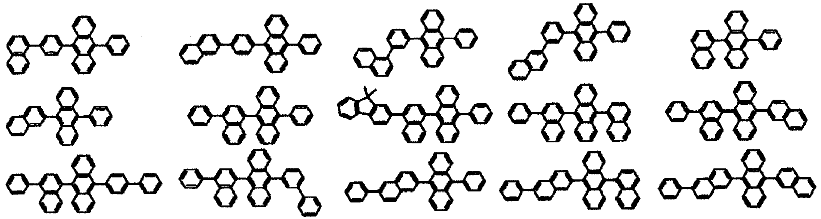

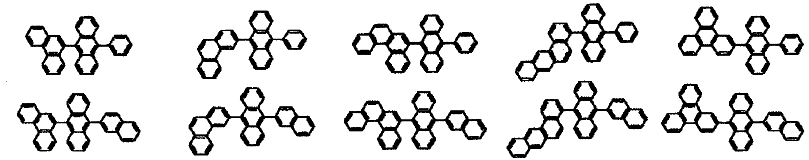

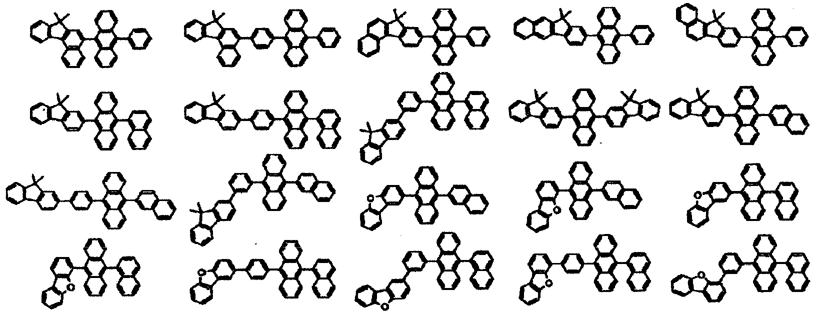

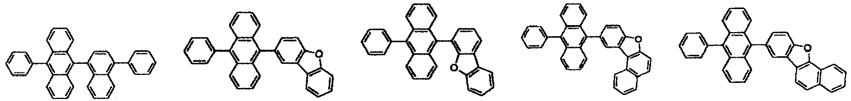

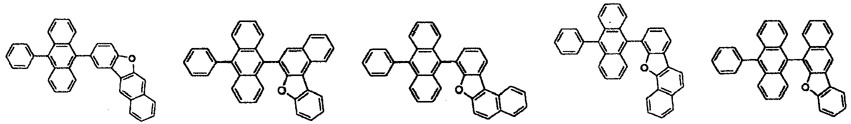

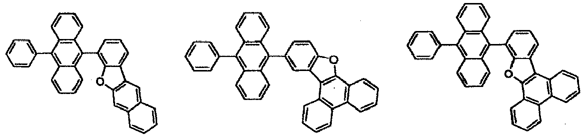

The polycyclic aromatic skeleton-containing compound that can be used as the host material of the emitting layer is a pyrene derivative represented by the following formula (6), for example. 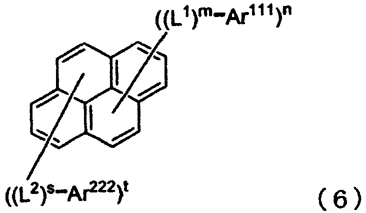
wherein in the formula (6), Ar¹¹¹ and Ar²²² are independently a substituted or unsubstituted aryl group including 6 to 30 ring carbon atoms;
L**¹** and L² are independently a substituted or unsubstituted divalent arylene group including 6 to 30 ring carbon atoms or a substituted or unsubstituted heterocyclic group including 6 to 30 ring atoms;
m is an integer of 0 to 1, n is an integer of 1 to 4, s is an integer of 0 to 1 and t is an integer of 0 to 3; and
L¹ or Ar¹¹¹ is bonded to any one of the 1^{st} to the 5^{th} positions of the pyrene and L² or Ar²²² is bonded to any one of the 6^{th} to 10^{th} positions of the pyrene.

L¹ and L² in the formula (6) are preferably a substituted or unsubstituted phenylene group, a substituted or unsubstituted biphenylene group, a substituted or unsubstituted naphthylene group, a substituted or unsubstituted terphenylene group, a substituted or unsubstituted fluorenylene group or a divalent aryl group composed of a combination of these substituents.

As the substituent, the same substituents as those in the "substituted or unsubstituted..." in the above formula (1) can be given. The substituent for L¹ and L² is preferably an alkyl group including 1 to 20 carbon atoms.

m in the formula (6) is preferably an integer of 0 to 1. n in the formula (6) is preferably an integer of 1 to 2. s in the formula (6) is preferably an integer of 0 to 1.

t in the formula (6) is preferably an integer of 0 to 2.

The aryl group of Ar¹¹¹ and Ar²²² is the same as the aryl group in the formula (1).

The aryl group is preferably a substituted or unsubstituted aryl group including 6 to 20 ring carbon atoms, more preferably a substituted or unsubstituted aryl group including 6 to 16 ring carbon atoms, and as preferable specific examples of the aryl group, a phenyl group, a naphthyl group, a phenanthryl group, a fluorenyl group, a biphenyl group, an anthryl group and a pyrenyl group can be given.

When the compound represented by the formula (1) is contained as a dopant, the content thereof is preferably 0.1 to 20 wt%, more preferably 1 to 10 wt%, relative to the total weight of the organic thin film layers containing the compound.

The compound represented by the formula (1) and an anthracene derivative or a pyrene derivative can be used, in addition to the emitting layer, in a hole-injecting layer, a hole-transporting layer, an electron-injecting layer and an electron-transporting layer.

In one aspect of the invention, as an organic EL device in which the organic thin film layers are composed of plural layers, one obtained by stacking layers in the following configurations can be given. (Anode/Hole-injecting layer/Emitting layer/Cathode), (Anode/Emitting layer/Electron-injecting layer/Cathode), (Anode/Hole-injecting layer/Emitting layer/Electron-injecting layer/Cathode), (Anode/Hole-injecting layer/Hole-transporting layer/Emitting layer/Electron-injecting layer/Cathode), etc.

In the organic EL device, by providing a plurality of organic thin film layers, deterioration of luminance or shortening in lifetime due to quenching can be prevented. If necessary, an emitting material, a doping material, a hole-injecting material or an electron-injecting material can be used in combination. Further, by using a doping material, luminance or luminous efficiency may be improved. The hole-injecting layer, the emitting layer and the electron-injecting layer may respectively be formed of two or more layers. In this case, in the case of the hole-injecting layer, a layer that injects holes from the electrode is referred to as a hole-injecting layer and a layer that receives electrons from the hole-injecting layer and transports the received holes to the emitting layer is referred to as a hole-transporting layer. Similarly, in the case of an electron-injecting layer, a layer that injects electrons from the electrode is referred to as an electron-injecting layer and a layer that receives electrons from the electron-injecting layer and transports the received electrons to the emitting layer is referred to as an electron-transporting layer. Each of these layers is used by selecting based on factors such as the energy level of the materials, heat resistance, adhesiveness with an organic layer or a metal electrode or the like.

As a material that can be used with the compound represented by the formula (1) other than the derivative represented by the formula (5), a fused polycyclic aromatic compound such as naphthalene, phenanthrene, rubrene, anthracene, tetracene, pyrene, perylene, chrysene, decacyclene, coronene, tetraphenylcyclopentadiene, pentaphenylcyclopentadiene, fluorene and spirofluorene, and derivatives thereof; an organic metal complex such as tris(8-quinolinato)aluminum; triarylamine derivatives, styrylamine derivatives, stilbene derivatives, coumarin derivatives, pyran derivatives, oxazone derivatives, benzothiazole derivatives, benzoxazole derivatives, benzimidazole derivatives, pyrazine derivatives, cinnamic acid ester derivatives, diketopyrrolopyrrole derivatives, acridone derivatives, quinacridone derivatives can be given, although not limited thereto.

A hole-injecting layer is a layer that comprises a substance having high hole-injecting properties. As a substance having high hole-injecting properties, molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, aromatic amine compound or a polymer compound (oligomer, dendrimer, polymer, or the like) or the like can also be used.

A hole-transporting layer is a layer that comprises a substance having high hole-transporting properties. In the hole-transporting layer, an aromatic amine compound, a carbazole derivative, an anthracene derivative or the like can be used. A polymer compound such as poly(N-vinylcarbazole) (abbreviation: PVK) or poly(4-vinyltriphenylamine) (abbreviation: PVTPA) can be used. However, other substances than those mentioned above can be used as long as it is a substance that exhibits high transporting properties for holes rather than for electrons. The layer that comprises a substance having high hole-transporting properties may be not only a single layer but also one obtained by stacking two or more layers that comprise the above-mentioned substances.

An electron-transporting layer is a layer that comprises a substance having high electron-transporting properties. In the electron-transporting layer, 1) a metal complex such as an aluminum complex, a beryllium complex, a zinc complex or the like; 2) a heterocyclic aromatic compound such as an imidazole derivative, a benzimidazole derivative, an azine derivative, a carbazole derivative, a phenanthroline derivative or the like; and 3) a polymer compound can be used.

An electron-injecting layer is a layer that comprises a substance having high electron-injecting properties. In the electron-injecting layer, an alkali metal such as lithium (Li), lithium fluoride (LiF), cesium fluoride (CsF), calcium fluoride (CaF₂) and lithium oxide (LiOx), an alkaline earth metal, or a compound thereof can be used.

In the organic EL device as one aspect of the invention, in the emitting layer, in addition to at least one selected from the compound represented by the formula (1), at least one selected from an emitting material, a doping material, a hole-injecting material, a hole-transporting material and an electron-transporting material may be contained in the same layer. Further, in order to improve stability of the organic EL device as one aspect of the invention against temperature, humidity, atmosphere or the like, it is possible to provide a protective layer on the surface of the device, and to protect the entire device with silicone oil, a resin or the like.

In the anode formed on the substrate, it is preferable to use a metal having a large work function (specifically, 4.0 eV or more), alloys, an electrically conductive compound, a mixture thereof, or the like. Specifically, indium oxide-tin oxide (ITO: Indium Tin Oxide), indium oxide-tin oxide containing silicon or silicon oxide, indium oxide-zinc oxide, tungsten oxide, indium oxide containing zinc oxide, graphene or the like can be given. In addition, gold (Au), platinum (Pt) or a nitride of a metal material (e.g. titanium nitride) or the like can be given.

In the cathode, it is preferable to use a metal having a small work function (specifically, 3.8 eV or less), alloys, an electrically conductive compound, a mixture thereof, or the like. As specific examples of the cathode material, an element belonging to Group 1 or Group 2 of the periodic table; that is an alkali metal such as lithium (Li) or cesium (Cs) and an alkaline earth metal such as magnesium (Mg) and alloys containing these (e.g. MgAg, AlLi) can be given.

The anode and the cathode may be formed of two or more layers, if necessary.

In the organic EL device, in order to realize efficient emission, it is desired that at least one surface be fully transparent in an emission wavelength region of the device. Further, it is desirable to allow the substrate to be transparent. A transparent electrode is set such that prescribed transparency is ensured by a deposition method, a sputtering method or the like by using the above-mentioned conductive materials. It is desirable that the electrode on the emission surface have light transmittance of 10% or more.

As the substrate, glass, quartz, plastics or the like can be used. A flexible substrate may be used. A flexible substrate is a substrate that can be bent. For example, a plastic substrate formed of polycarbonate and polyvinyl chloride, or the like can be given.

Each layer of the organic EL device can be formed by any of a dry film-forming method such as vacuum deposition, sputtering, plasma and ion plating or a wet film-forming method such as spin coating, dipping and flow coating. The film thickness is not particularly restricted, but it is required to set to an appropriate thickness. If the thickness is too large, it is required to apply a large voltage in order to obtain a prescribed optical output, thus leading to a poor efficiency. If the film thickness is too small, pin holes or the like are generated, and a sufficient luminance cannot be obtained even if an electrical field is applied. Normally, the film thickness is preferably in a range of 5 nm to 10 µm, with a range of 10 nm to 0.2 µm being further preferable.

In the case of a wet film-forming method, a thin film is formed by dissolving or dispersing a material forming each layer in an adequate solvent such as ethanol, chloroform, tetrahydrofuran and dioxane. Any of these solvents may be used.

As a solution suited to such wet film-forming method, a solution that contains a material for an organic EL device that contains the compound represented by the formula (1) and a solvent can be used.

It is preferred that the material for an organic EL device contain a host material and a dopant material, and the dopant material be a compound represented by the formula (1) and the host material be at least one selected from compounds represented by the formula (5).

In each of the organic thin film layers, in order to improve film-forming properties, to prevent generation of pin holes in a film, or other problems, an adequate resin or additive may be used.

The organic EL device as one aspect of the invention can be used in an electronic apparatus. Specifically, it can be used in a planer emitting body such as a flat panel display of a wall-hanging TV, a copier, a printer, back light of a liquid crystal display, a light source of an instrument or the like, a display board, a marker lamp or the like. The compound according to one aspect of the invention can be used not only in an organic EL device but also in the field of an electrophotographic photoreceptor, a photoelectric conversion device, a solar cell, an image sensor or the like.

### EXAMPLES

Hereinbelow, the invention will be described in more detail in accordance with the following examples, which should not be construed as limiting the scope of the invention.

### Synthesis Example 1 Synthesis of compound 1

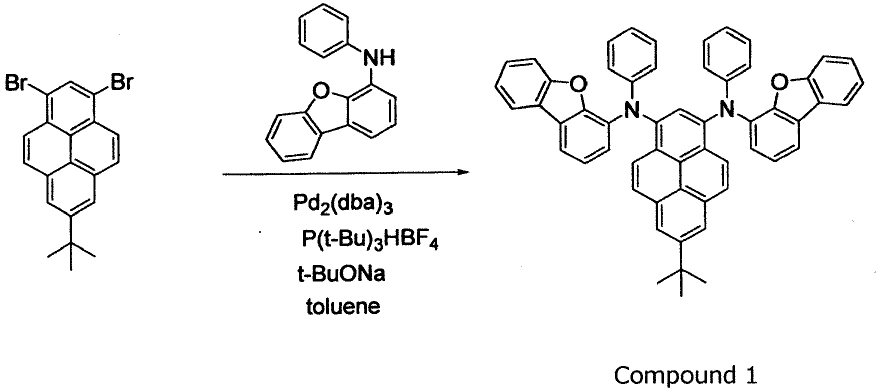

In an argon atmosphere, 4.00g of 1,3-dibromo-7-t-butylpyrene, 5.48g of 4-anilinodibenzofuran, 0.220g of tris(dibenzylideneacetone)dipalladium(0), 0.139g of tri-t-butylphosphonium tetrafluoroborate, 2.96g of sodium-t-butoxide and 100 mL of toluene were put in a flask, followed by stirring under reflux with heating for 5 hours. After cooling to room temperature, unsoluble matters were removed by filtration. The reaction solution was concentrated, and residues were purified by silica gel column chromatography, whereby 5.37g of pale yellow solids were obtained. As a result of mass spectroscopy, the resulting product was found to be compound 1 as an intended product, and had an m/e value of 772 relative to a molecular weight of 772.31.

### Synthesis Example 2 Synthesis of compound 2

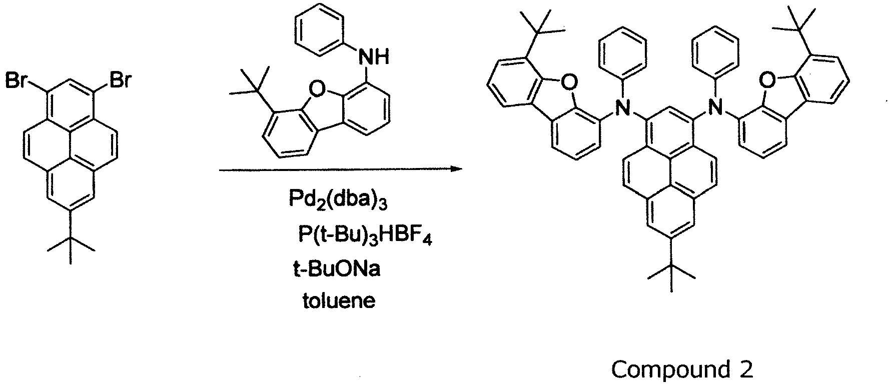

Synthesis was conducted in the same manner as in Synthesis Example 1, except that 4-anilino-6-t-butyldibenzofuran was used instead of 4-anilinodibenzofuran. As a result of mass spectroscopy, the resulting product was found to be compound 2 as an intended product, and had an m/e value of 884 relative to a molecular weight of 884.43.

### Synthesis Example 3 Synthesis of compound 3

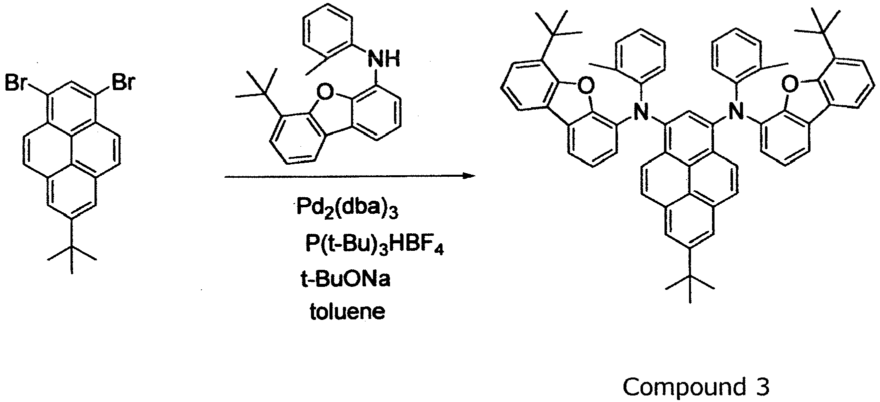

Synthesis was conducted in the same manner as in Synthesis Example 1, except that 4-(N-o-tolyamino)-6-t-dibenzofuran was used instead of 4-anilinodibenzofuran. As a result of mass spectroscopy, the resulting product was found to be compound 3 as an intended product, and had an m/e value of 912 relative to a molecular weight of 912.47.

### Synthesis Example 4 Synthesis of compound 4

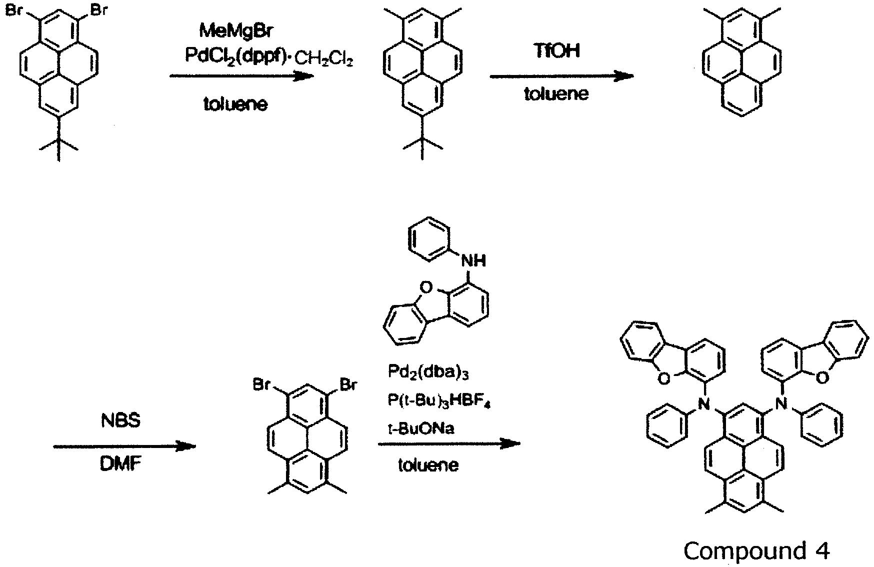

### (4-1) Synthesis of 7-t-butyl-1,3-dimethylpyrene

In an argon atmosphere, 10.4g of 1,3-dibromo-7-t-butylpyrene, 0.408g of [1,1'-bis(diphenylphosphino)ferrocene]palladium (II) dichloride dichloromethane adduct and 100 mL of toluene were put in a flask. While stirring, 81.5 mL of a tetrahydrofuran solution (0.92M) of methyl magnesium bromide was added, followed by stirring under reflux with heating for 2.5 hours. After cooling to room temperature, water and methylene chloride were added and an organic layer was collected. The organic layer was concentrated, and the residues were purified by silica gel column chromatography, whereby 6.07g of white solids were obtained. As a result of mass spectroscopy, the resulting product was found to be 7-t-butyl-1,3-dimethylpyrene and had an m/e value of 286 relative to a molecular weight of 286.1.

### (4-2) Synthesis of 1,3-dimethylpyrene

A mixed solution of 30 mL of trifluoromethanesulfonic acid and 270 mL of toluene was added to 5.00g of 7-t-butyl-1,3-dimethylpyrene, and the resultant was stirred with heating in an argon atmosphere at 80°C for 2 hours. After cooling to room temperature, the reaction solution was poured to 150 mL of water, and an organic layer was collected. The organic layer was concentrated, and the residues were purified by silica gel column chromatography, whereby 3.26g of white solids were obtained. As a result of mass spectroscopy, the resulting product was found to be a compound as an intended product had an m/e value of 230 relative to a molecular weight of 230.3.

### (4-3) Synthesis of 1,3-dibromo-6,8-dimethylpyrene

In an argon atmosphere, 2.00g of 1,3-dimethylpyrene, 3.09g of N-bromosuccinimide and 80 mL of dimethylformamide were put in a flask, followed by stirring at room temperature for 9 hours. After the reaction, deposited solids were removed by filtration, whereby 2.50g of white solids were obtained. As a result of mass spectroscopy, the resulting product was found to be 1,3-dibromo-6,8-dimethylpyrene, and an m/e value of 388 relative to a molecular weight of 388.10.

### (4-4) Synthesis of compound 4

Synthesis was conducted in the same manner as in Synthesis Example 1, except that 1,3-dibromo-6,8-dimethylpyrene synthesized in (4-3) was used instead of 1,3-dibromo-7-t-butylpyrene. As a result of mass spectroscopy, the resulting product was found to be compound 4 as an intended product and had an m/e value of 744 relative to a molecular weight of 744.3.

### (Production and evaluation of organic EL device)

### Example 1

A130 nm-thick glass substrate with ITO transparent electrode lines (manufactured by GEOMATEC Co., Ltd.) was subjected to ultrasonic cleaning for 5 minutes in isopropyl alcohol, and then subjected to UV-ozone cleaning for 30 minutes.

The cleaned glass substrate with the transparent electrode lines was mounted in a substrate holder of a vacuum vapor deposition apparatus. First, compound HAT (hexaazatriphenylene) was deposited on the surface where transparent electrode lines were formed so as to cover the transparent electrode, thereby to form a 5 nm-thick HAT film.

Subsequently, compound HT-1 was deposited on the HAT film, whereby a 80 nm-thick HT-1 film was formed.

Then, on the HT-1 film, compound HT-2 was deposited, whereby a 10 nm-thick HT-2 film was formed.

Subsequently, on the HT-2 film, compound BH-1 (host material) and compound 1 (dopant material) were co-deposited, whereby a 25 nm-thick thin film was formed. At this time, deposition was conducted such that the amount of compound 1 became 4% in terms of mass ratio relative to the total mass of the compound BH-1 and the compound 1.

Subsequently, compound ET-1 was deposited on this thin film, whereby a 10 nm-thick ET-1 film was formed.

Then, on the ET-1 film, compound ET-2 was deposited, whereby a 15 nm-thick ET-2 film was formed.

Subsequently, on the ET-2 film, LiF was deposited, whereby a 1 nm-thick LiF film was formed.

Finally, metal Al was deposited on the LiF film, whereby a 80 nm-thick Al film was formed.

In the above-mentioned way, an organic EL device was produced.

### (Evaluation of organic EL device)

To the obtained organic EL device, a voltage was applied such that the current density became 10 mA/cm². EL emission spectrum was measured by means of a spectroradiometer (CS-1000: manufactured by Konica Minolta, Inc.). From the resulting spectral emission luminance spectrum, the external quantum efficiency EQE (%) was calculated. The emission peak wavelength λₑₘ (nm) and the external quantum efficiency EQE (%) are shown in Table 1.

### Examples 2 to 3 and Comparative Example 1

Organic EL devices were produced and evaluated in the same manner as in Example 1, except that compounds shown in Table 1 were used instead of compound 1. The results are shown in Table 1.

Compounds used in Examples and Comparative Example are shown below.

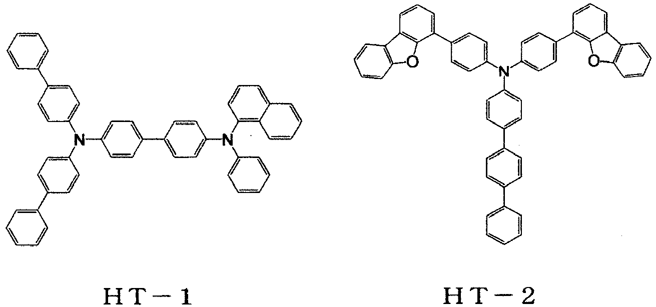

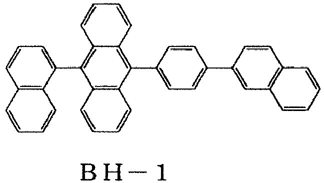

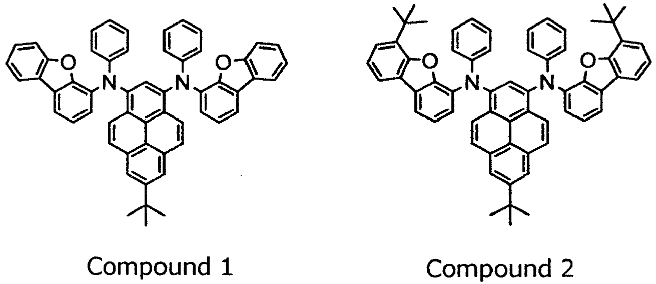

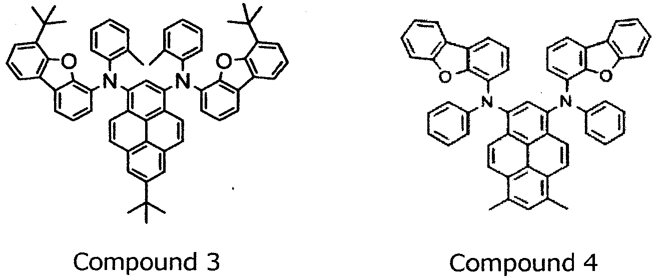

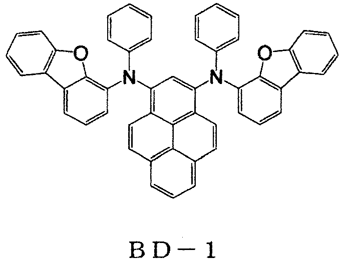

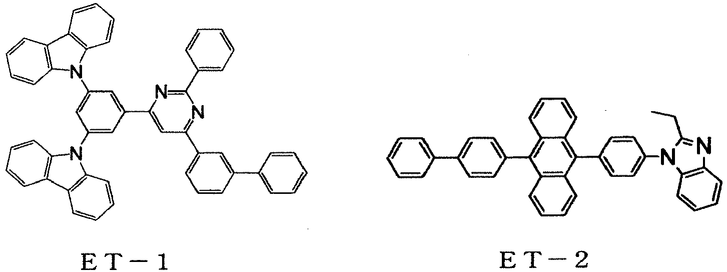

**Table 1**

| | Dopant material | λₑₘ (nm) | EQE (%) |
|---|---|---|---|
| Example 1 | Compound 1 | 449 | 6.7 |
| Example 2 | Compound 2 | 450 | 7.3 |
| Example 3 | Compound 3 | 452 | 7.7 |
| Example 4 | Compound 4 | 452 | 6.6 |
| Comp. Ex. 1 | Compound BD-1 | 453 | 5.5 |

From Table 1, it can be understood that, by using the compound of the invention in an organic EL device, it is possible to allow the emission wavelength to be short, and to realize excellent efficiency.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The specification of a Japanese application on the basis of which the present application claims Paris Convention priority is incorporated herein by reference in its entirety.

## Claims

1. A compound represented by the following formula (1): 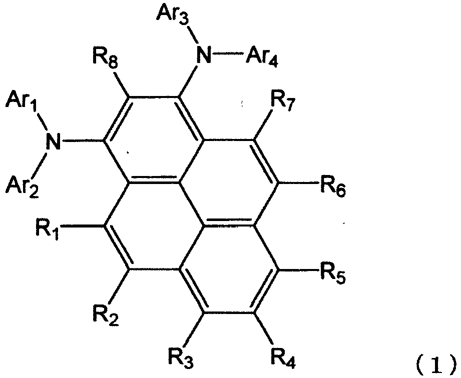
wherein in the formula (1), R₁ to R₈ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms;
at least one of R₁ to R₈ is a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms;
Ar₁ to Ar₄ are independently a substituted or unsubstituted aryl group including 6 to 30 ring carbon atoms or a substituted or unsubstituted heteroaryl group including 5 to 30 ring atoms; and
at least one of Ar₁ to Ar₄ is a group represented by the following formula (2): 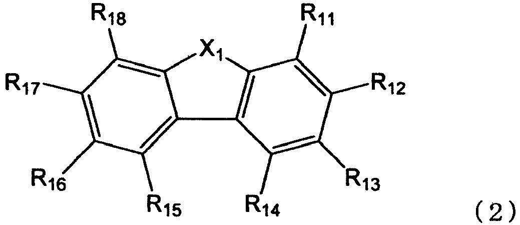
wherein in the formula (2), R₁₁ to R₁₈ are independently a single bond that is bonded to N in the formula (1), a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms;
among R₁₁ to R₁₈, any adjacent two may be bonded to each other to form a ring; and
X₁ is an oxygen atom or a sulfur atom.

2. The compound according to claim 1, wherein at least two of Ar₁ to Ar₄ are groups represented by the formula (2).

3. The compound according to claim 1 or 2, wherein Ar₁ and Ar₃ are groups represented by the formula (2).

4. The compound according to any one of claims 1 to 3, wherein R₁₁ is a single bond that is bonded to N in the formula (1).

5. The compound according to any one of claims 1 to 4, wherein at least one of R₃ to R₅ is a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms.

6. The compound according to claim 5, wherein R₄ is a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms.

7. The compound according to claim 5, wherein R₃ and R₅ are independently a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms or a substituted or unsubstituted alkoxy group including 1 to 15 carbon atoms.

8. The compound according to any one of claims 1 to 7, wherein at least one of R₁ to R₈ is a substituted or unsubstituted alkyl group including 1 to 15 carbon atoms, a substituted or unsubstitued alkylsilyl group including 3 to 45 carbon atoms or a substituted or unsubstituted arylsilyl group including 8 to 50 ring carbon atoms.

9. The compound according to any one of claims 1 to 8, wherein at least one of R₁ to R₈ is a substituted or unsubstituted alkyl group including 1 to 6 carbon atoms.

10. The compound according to any one of claims 1 to 9, wherein R₁₁ is a single bond that is bonded to N in the formula (1) and R₁₈ is a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted aryl group including 6 to 30 ring carbon atoms or a substituted or unsubstituted heteroaryl group including 5 to 30 ring atoms.

11. The compound according to any one of claims 1 to 10, wherein the group represented by the formula (2) is a group represented by the following formula (3) or the following formula (4): 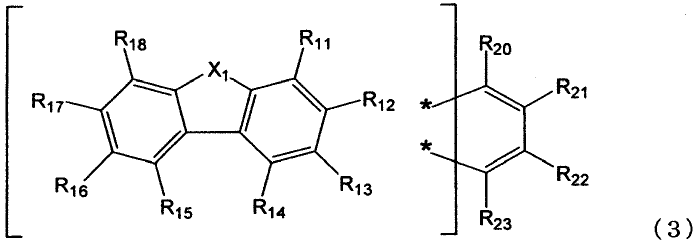
wherein in the formula (3), R₁₁ to R₁₈ and X₁ are the same as defined in the formula (2),
R₂₀ to R₂₃ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms; and
* is a bonding position to any adjacent two of R₁₁ to R₁₈. 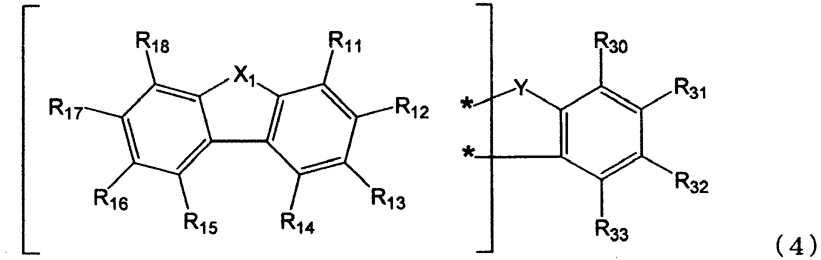
wherein in the formula (4), R₁₁ to R₁₈ and X₁ are the same as defined in the formula (2);
Y is an oxygen atom, a sulfur atom or CR₃₄R₃₅;
R₃₀ to R₃₅ are independently a hydrogen atom, a halogen atom, a cyano group, a substituted or unsubstituted alkyl group including 1 to 15 carbon atoms, a substituted or unsubstituted alkylsilyl group including 3 to 45 carbon atoms, a substituted or unsubstituted arylsilyl group including 8 to 50 ring carbon atoms, a substituted or unsubstituted alkoxy group including 1 to 15 carbon atoms, a substituted or unsubstituted aryloxy group including 6 to 30 ring carbon atoms, a substituted or unsubstituted alkylthio group including 1 to 15 carbon atoms, a substituted or unsubstituted arylthio group including 6 to 30 ring carbon atoms, a substituted or unsubstituted aryl group including 6 to 30 ring carbon atoms or a substituted or unsubstituted heteroaryl group including 5 to 30 ring atoms; and
* is a bonding position to any adjacent two of R₁₁ to R₁₈.

12. The compound according to any one of claims 1 to 11 which is a material for an organic electroluminescence device.

13. The compound according to any one of claims 1 to 11 which is an emitting material for an organic electroluminescence device.

14. The compound according to any one of claims 1 to 11 which is a dopant material for an organic electroluminescence device.

15. An organic electroluminescence device comprising a cathode and an anode and one or more organic thin film layers including at least an emitting layer being disposed between the cathode and the anode, wherein
at least one layer of the organic thin film layers comprises the compound according to any one of claims 1 to 11 singly or as a component of a mixture.

16. The organic electroluminescence device according to claim 15, wherein the emitting layer comprises the compound.

17. The organic electroluminescence device according to claim 16, wherein the compound is a dopant material.

18. The organic electroluminescence device according to any one of claims 15 to 17, wherein at least one of the organic thin film layers further comprises the compound represented by the following formula (5): 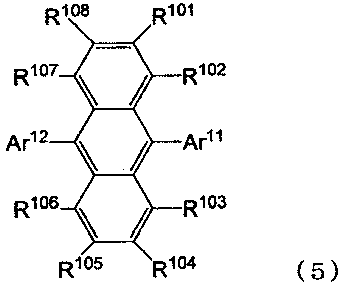
wherein in the formula (5), Ar¹¹ and Ar¹² are independently a substituted or unsubstituted monocyclic ring group including 5 to 50 ring atoms or a substituted or unsubstituted fused cyclic group including 8 to 50 ring atoms; and
R¹⁰¹ to R¹⁰⁸ are independently a hydrogen atom, a substituted or unsubstituted monocyclic ring group including 5 to 50 ring atoms, a substituted or unsubstituted fused cyclic group including 8 to 50 ring atoms, a group composed of a combination of the monocyclic ring group and the fused cyclic group, a substituted or unsubstituted alkyl group including 1 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted aralkyl group including 7 to 50 carbon atoms, a substituted or unsubstituted aryloxy group including 6 to 50 ring carbon atoms, a substituted or unsubstituted silyl group, a halogen atom or a cyano group.

19. The organic electroluminescence device according to claim 18, wherein in the formula (5), Ar¹¹ and Ar¹² are independently a substituted or unsubstituted fused cyclic group including 8 to 50 ring atoms.

20. The organic electroluminescence device according to claim 18, wherein in the formula (5), one of Ar¹¹ and Ar¹² is a substituted or unsubstituted monocyclic ring group including 5 to 50 ring atoms and the other of Ar¹¹ and Ar¹² is a substituted or unsubstituted fused cyclic group including 8 to 50 ring atoms.

21. The organic electroluminescence device according to claim 20, wherein in the formula (5), Ar¹¹ is an unsubstituted phenyl group or a phenyl group substituted with a monocyclic ring group or a fused cyclic group, and Ar¹² is a naphthyl group, a phenanthryl group, a benzanthryl group, a fluorenyl group or a dibenzofuranyl group.

22. The organic electroluminescence device according to claim 20, wherein in the formula (5), Ar¹¹ is an unsubstituted phenyl group and Ar¹² is a substituted or unsubstituted fused cyclic group including 8 to 50 ring atoms.

23. The organic electroluminescence device according to claim 18, wherein in the formula (5), Ar¹¹ and Ar¹² are independently a substituted or unsubstituted monocyclic ring group including 5 to 50 ring atoms.

24. The organic electroluminescence device according to claim 23, wherein in the formula (5), Ar¹¹ and Ar¹² are independently a substituted or unsubstituted phenyl group.

25. The organic electroluminescence device according to claim 24, wherein in the formula (5), Ar¹¹ is an unsubstituted phenyl group and Ar¹² is a phenyl group substituted with a monocyclic ring group or a fused cyclic group.

26. The organic electroluminescence device according to claim 24, wherein in the formula (5), Ar¹¹ and Ar¹² are independently a phenyl group substituted with a monocyclic ring group or a fused cyclic group.

27. An electronic apparatus that is provided with the organic electroluminescence device according to any one of claims 15 to 26.
